(19) Europäisches Patentamt
European Patent Office
Office européen des brevets

(11) Veröffentlichungsnummer: **0 399 294 B1**

(12) # EUROPÄISCHE PATENTSCHRIFT

(45) Veröffentlichungstag der Patentschrift: **02.02.94**

(51) Int. Cl.[5]: **C07D 249/12**, C07D 405/12, C07D 405/04, C07D 405/06, A01N 47/38

(21) Anmeldenummer: **90108779.1**

(22) Anmeldetag: **10.05.90**

Die Akte enthält technische Angaben, die nach dem Eingang der Anmeldung eingereicht wurden und die nicht in dieser Patentschrift enthalten sind.

(54) **Substituierte Triazolinone.**

(30) Priorität: **24.05.89 DE 3916930**
**06.01.90 DE 4000234**

(43) Veröffentlichungstag der Anmeldung:
**28.11.90 Patentblatt 90/48**

(45) Bekanntmachung des Hinweises auf die Patenterteilung:
**02.02.94 Patentblatt 94/05**

(84) Benannte Vertragsstaaten:
**BE CH DE FR GB IT LI NL**

(56) Entgegenhaltungen:
**EP-A- 0 294 666**
**DE-A- 2 416 814**
**FR-A- 2 546 887**
**GB-A- 2 194 534**

**CHEMICAL ABSTRACTS, Band 103, Nr. 3, 22. Juli 1985, Seite 571, Zusammenfassung-Nr. 22524w, Columbus, Ohio, US; F. MALBEC et al.: "Derivatives of 2, 4-dihydro-1,2,4-triazole-3-thione and 2-amino-1,3,4-thiadiazole from thiosemicarbazones of esters"**

(73) Patentinhaber: **BAYER AG**

**D-51368 Leverkusen(DE)**

(72) Erfinder: **Müller, Klaus-Helmut, Dr.**
**Bockhackstrasse 55**
**D-4000 Düsseldorf 13(DE)**
Erfinder: **König, Klaus, Dr.**
**Zum Hahnenberg 40**
**D-5068 Odenthal(DE)**
Erfinder: **Findeisen, Kurt, Dr.**
**Duenfelder Strasse 28**
**D-5090 Leverkusen 1(DE)**
Erfinder: **Santel, Hans-Joachim, Dr.**
**Grünstrasse 9a**
**D-5090 Leverkusen 1(DE)**
Erfinder: **Lürssen, Klaus, Dr.**
**August-Kirspel-Strasse 145**
**D-5060 Bergisch-Gladbach 2(DE)**
Erfinder: **Schmidt, Robert R., Dr.**
**Im Waldwinkel 110**
**D-5060 Bergisch-Gladbach 2(DE)**
Erfinder: **Dutzmann, Stefan, Dr.**
**Leinenweberweg 33**
**D-4000 Düsseldorf 13(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, Band 80, Nr. 5, 4. Februar 1974, Seite 417, Zusammenfassung-Nr. 27172w, Columbus, Ohio, US; R. GRASHEY et al.: "Phenylazo- and phenylhydrazino-1,3,4-thiadiazoles"

CHEMICAL ABSTRACTS, Band 94, Nr. 3, 19. Januar 1981, Seite 434, Zusammenfassung-Nr. 15645d, Columbus, Ohio, US; A. IKIZLER et al.: "Reactions of ester ethoxycarbonylhydrazones with some amine type compounds"

CHEMICAL ABSTRACTS, Band 83, Nr. 25, 22. Dezember 1975, Seite 346, Zusammenfassung-Nr. 205712u, Columbus, Ohio, US; R. ESMAIL et al.:" Heterocyclic compounds from urea derivatives. XXII. Thiobenzoylated carbonohydrazides and their cyclization"

CHEMICAL ABSTRACTS, Band 95, Nr. 13, 28. September 1981, Seite 692, Zusammenfassung-Nr. 115397p, Columbus, Ohio, US; A.A. EL-BARBARY et al.: "Reaction of 5-aryl-2-thiono-1,3,4-oxadiazoles with some amino- and halo compounds"

CHEMICAL ABSTRACTS, Band 76, Nr. 15, 10. April 1972, seite 414, Zusammenfassung-Nr. 85755s, Columbus, Ohio, US; H.G.O. BECKER et al.:"Relay synthesis of heterocycles. X. Preparation of 4-alkylamino-1,2,4-triazol-3-ones"

CHEMICAL ABSTRACTS, Band 110, Nr. 24, 12. Juni 1989, Seite 665, Zusammenfassung-Nr. 222506y, Columbus, Ohio, US; A. YOSHIDA et al.: "Silver complex diffusion-transfer photographic method providing fine graded images"

CHEMICAL ABSTRACTS, Band 97, Nr. 1, 5. Juli 1982, Seite 603, Zusammenfassung-Nr. 6227d, Columbus, Ohio, US; E. AYCA et al.: " Preparation of 3-alkyl(aryl)-4-alkylamino(arylamino)-delta2-1,2,-4-triazolin-5-ones"

CHEMICAL ABSTRACTS, Band 88, Nr. 17, 24. April 1978, Seite 461, Zusammenfassung-Nr. 120169a, Columbus, Ohio, US; M. AOUIAL et al.: "Fragmentation under electron impact of 4,5-diamino-1,2,4-triazoles and 4-amino-5-mercapto-1,2,4-triazoles"

CHEMICAL ABSTRACTS, Band 90, Nr. 24, 11. Juni 1979, Seite 594, Zusammenfassung-Nr. 195585h, Columbus, Ohio, US;E. KANEDA et al.: "Silver halide color photographic photosensitive materials"

PATENT ABSTRACTS OF JAPAN, Band 2, Nr. 24, 16. Februar 1978, Seite C77, NIPPON SODA K.K.: "Triazoline derivatives, method of preparing the same, and herbicides"

## Beschreibung

Die Erfindung betrifft neue substituierte Triazolinone, mehrere Verfahren zu ihrer Herstellung und ihre Verwendung als Herbizide und Fungizide.

Es ist bekannt, daß bestimmte Stickstoffheterocyclen wie beispielsweise 4-Amino-3-methyl-6-phenyl-1,2,4-triazin-5-on (vgl. DE-OS 23 64 474) oder 4-Amino-1-(N-phenylcarbamoyl)-3-methyl-triazolin-5-on (vgl. EP-A 294666) herbizide Eigenschaften besitzen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinone, wie beispielsweise das 1-(N,N-Dimethylcarbamoyl)-3-phenyl-4-amino-1,2,4-triazolin-5-on bekannt (vergl. J.Heterocycl. Chem. 17, 1691-1696 [1980]; Org. Mass. Spectrom. 14, 369-378 [1979]).
Über eine Wirksamkeit dieser vorbekannten Triazolinone als Herbizide ist bisher nichts bekannt.

Es wurden neue substituierte Triazolinone der allgemeinen Formel (I),

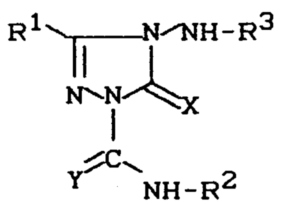

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für Tetrahydrofuranyl oder für Tetrahydrofuranylalkyl mit gegebenenfalls 1 bis 4 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach oder mehrfach gleich oder verschieden substituiertes Cycloalkyl, Cycloalkylalkyl, Cycloalkenyl oder Cycloalkenylalkvl mit jeweils 3 bis 8 Kohlenstoffatomen im Cycloalkylbzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4

Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter $-O-CH_2-O-$ Gruppe steht,

$R^3$ für $C_1$-$C_8$-Alkyl, $C_2$-$C_8$-Alkenyl, $C_3$-$C_6$-Cycloalkyl oder Phenyl-$C_1$-$C_4$-alkyl steht,
X für Sauerstoff oder Schwefel steht und
Y für Sauerstoff oder Schwefel steht,

gefunden.

Die Verbindungen der Formel (I) können gegebenenfalls in Abhängigkeit von der Art der Substituenten $R^1$, $R^2$ und $R^3$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

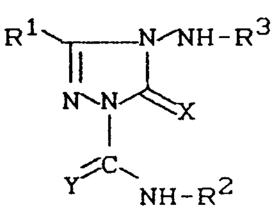

(I)

in welcher
$R^1$, $R^2$, $R^3$, X und Y die oben angegebenen Bedeutungen haben
erhält, wenn man

(a) 1H-Triazolinone der allgemeinen Formel (II)

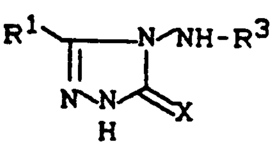

(II)

in welcher
$R^1$, $R^3$ und X die oben angegebene Bedeutung haben,
mit Iso(thio)cyanaten der allgemeinen Formel (III)

$R^2-N=C=Y$ (III)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder wenn man

(b) 1H-Triazolinone der allgemeinen Formel (IV)

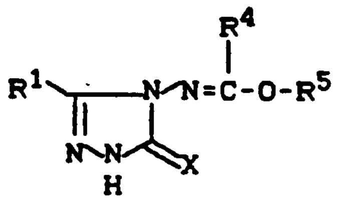

(IV)

in welcher

$R^1$ und X die oben angegebene Bedeutung haben und

$R^4$ und $R^5$ unabhängig voneinander für $C_1$-$C_8$-Alkyl, $C_2$-$C_8$-Alkenyl, $C_2$-$C_8$-Alkinyl, Aryl oder Arylalkyl mit jeweils 6 bis 10 Kohlenstoffatomen im Arylteil und gegebenenfalls 1 bis 6 Kohlenstoffatomen im geradkettigen oder verzweigten Alkylteil oder für Cycloalkyl mit jeweils 3 bis 7 Kohlenstoffatomen, stehen, sowie $R^4$ auch für Wasserstoff steht,

mit Iso(thio)cyanaten der allgemeinen Formel (III)

$R^2$-N=C=Y (III)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt

und die so erhaltenen Verbindungen der allgemeinen Formel (V)

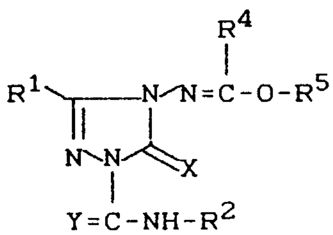

(V)

in welcher

$R^1$, $R^2$, $R^4$, $R^5$, X und Y die oben angegebene Bedeutung haben,

in einem zweiten Reaktionsschritt mit einem Reduktionsmittel der Formel (VI) oder (VII)

$MBH_4$ (VI)

$MAlH_4$ (VII)

in welchem

M für ein Alkalimetallatom steht,

gegebenenfalls in Gegenwart eines Verdünnungsmittels zu den Verbindungen der allgemeinen Formel (VIII)

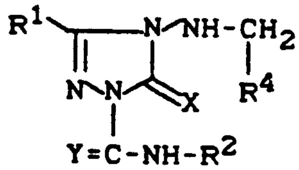

(VIII)

in welcher

$R^1$, $R^2$, $R^4$, X und Y die oben angegebene Bedeutung haben,

umsetzt,

oder wenn man

(c) 1-Carbamoyltriazolinone der allgemeinen Formel (IX)

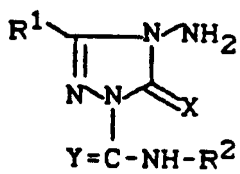

(IX)

in welcher

$R^1$, $R^2$, X und Y die oben angegebene Bedeutung haben,

mit Orthocarbonsäureestern dar allgemeinen Formel (X)

$R^4$-C($OR^5$)$_3$ (X)

in welcher

$R^4$ und $R^5$ die oben angegebene Bedeutung haben,

gegebenenfalls in Gegenwart eines Verdünnungsmittels und gegebenenfalls in Gegenwart eines Reaktionshilfsmittels zu Verbindungen der allgemeinen Formel (V)

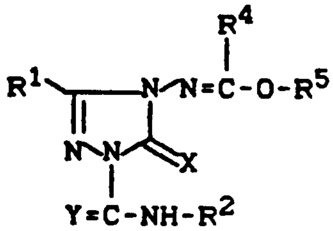

(V)

in welcher

$R^1$, $R^2$, $R^4$, $R^5$, X und Y die oben angegebene Bedeutung haben,

umsetzt und diese Verbindungen der Formel (V) in einem zweiten Reaktionsschritt mit einem Reduktionsmittel der Formel (VI) oder (VII)

$MBH_4$ (VI)

$MAlH_4$ (VII)

in welchem

M für ein Alkalimetallatom steht,

gegebenenfalls in Gegenwart eines Verdünnungsmittels zu den Verbindungen der allgemeinen Formel (VIII)

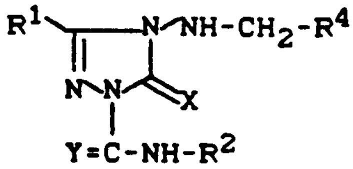

(VIII)

in welcher

$R^1$, $R^2$, $R^4$, X und Y die oben angegebene Bedeutung haben,

umsetzt,

oder wenn man

(d) 1H-Triazolinone der allgemeinen Formel (II)

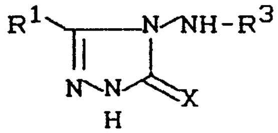

(II)

in welcher

$R^1$, $R^3$ und X die oben angegebene Bedeutung haben,

mit (Thio)Chlorameisensäureestern der allgemeinen Formel (XI)

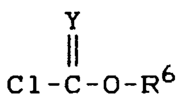

(XI)

in welcher

$R^6$ für $C_1$-$C_8$-Alkyl, Aryl oder Aralkyl mit jeweils 6 bis 10 Kohlenstoffatomen im Arylteil und gegebenenfalls 1 bis 6 Kohlenstoffatomen im geradkettigen oder verzweigten Alkylteil steht und

Y die oben angegebene Bedeutung hat,

gegebenenfalls in Gegenwart eines Verdünnungsmittels und gegebenenfalls in Gegenwart eines Reaktionshilfsmittels umsetzt und die so erhältlichen Triazolinone der allgemeinen Formel (XII)

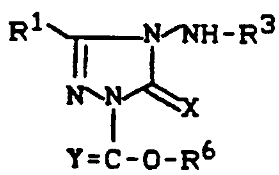

(XII)

in welcher

$R^1$, $R^3$, $R^6$, X und Y die oben angegebene Bedeutung haben,

in einer anschließenden 2. Stufe mit Aminen der allgemeinen Formel (XIII)

$R^2$-$NH_2$ (XIII)

in welcher

$R^2$ die oben angegebene Bedeutung hat,

gegebenenfalls in Gegenwart eines Verdünnungsmittels und gegebenenfalls in Gegenwart eines Reaktionshilfsmittels zu Verbindungen der Formel (I) umsetzt,
oder wenn man
(e) 1H-Triazolinone der allgemeinen Formel (II)

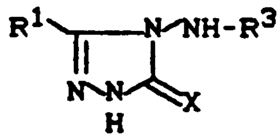

(II)

in welcher

$R^1$, $R^3$ und X die oben angegebene Bedeutung haben,

mit (Thio)Urethanen der allgemeinen Formel (XIV)

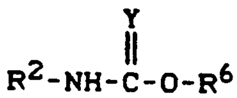

(XIV)

in welcher

$R^2$, $R^6$ und Y die oben angegebene Bedeutung haben,

gegebenenfalls in Gegenwart eines Verdünnungsmittels und gegebenenfalls in Gegenwart eines Reaktionshilfsmittels umsetzt,
oder wenn man
(f) Triazolinon-Hydrazone der allgemeinen Formel (XV)

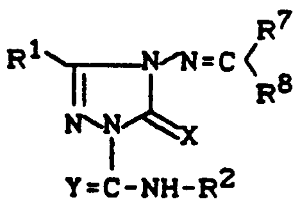

(XV)

in welcher

$R^1$, $R^2$, X und Y die oben angegebene Bedeutung haben und,

$R^7$ und $R^8$ jeweils unabhängig voneinander für Wasserstoff, Alkyl, Alkenyl, Alkinyl, Halogenalkyl, Halogenalkenyl, Cycloalkyl, Aryl, Aralkyl oder Halogenalkyl stehen,

mit Reduktionsmitteln gegebenenfalls in Gegenwart von Katalysatoren und gegebenenfalls in Gegenwart von Verdünnungsmitteln umsetzt.

Schließlich wurde gefunden, daß die neuen substituierten Triazolinone der allgemeinen Formel (I) ausgezeichnete herbizide und fungizide Eigenschaften besitzen.

Überraschenderweise zeigen die erfindungsgemäßen substituierten Triazolinone der allgemeinen Formel (I) eine erheblich höhere herbizide Potenz gegenüber Problemunkräutern als die aus dem Stand der Technik bekannten Stickstoffheterocyclen, wie beispielsweise das 4-Amino-3-methyl-6-phenyl-1,2,4-triazin-5-on oder 4-Amino-1-(N-phenylcarbamoyl)3-methyl-triazolin-5-on, welche chemisch und wirkungsmäßig naheliegende Verbindungen sind.

In den Definitionen stehen die aromatischen Reste wie beispielsweise Aryl, Aryloxy, Aralkyl vorzugsweise für Phenyl oder Naphthyl, insbesondere für Phenyl. Die aliphatischen Kohlenstoffketten sind, auch wenn es nicht ausdrücklich angegeben ist, jeweils geradkettig oder verzweigt.

Der Substituent Heterocyclylalkyl in $R^2$ bedeutet vorzugsweise Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen - insbesondere Stickstoff, Sauerstoff und/oder Schwefel - im Heterocyclylteil, wobei der

Heterocyclylteil einfach oder mehrfach, insbesondere einfach, zweifach oder dreifach, gleich oder verschieden substituiert sein kann mit Halogen, Cyano, Nitro sowie $C_1$-$C_5$-Alkyl, $C_1$-$C_5$-Alkoxy, $C_1$-$C_5$-Alkylthio, Halogen-$C_1$-$C_5$-alkyl, Halogen-$C_1$-$C_5$-alkoxy, Halogen-$C_1$-$C_5$-alkyl oder $C_1$-$C_5$-Alkoxycarbonyl. Der Heterocyclylteil kann insbesondere substituiert sein mit Fluor, Chlor, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio. Besonders bevorzugt steht der Substituent Heterocyclylalkyl in $R_2$ für im Heterocyclylteil gegebenenfalls ein bis dreifach, gleich oder verschieden substituiertes Heterocyclylmethyl, Heterocyclylpropyl oder Heterocyclylethyl, wobei als Heterocyclen jeweils infrage kommen:

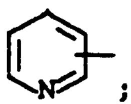 ; 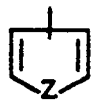 ; 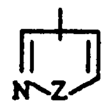 ; 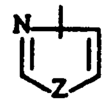 ;

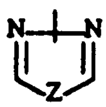 ; 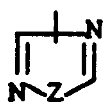 ; 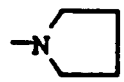 ; 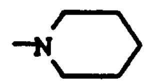 ;

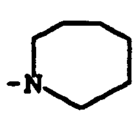 ; 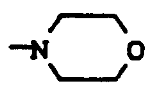 oder 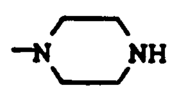

wobei Z jeweils für Sauerstoff oder Schwefel steht und wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio.

Die erfindungsgemäßen substituierten Triazolinone sind durch die Formel (I) allgemein definiert, Bevorzugt sind Verbindungen der Formel (I), bei welchen

6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkylbzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 2 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und

gegebenenfalls 1 bis 9 gleichen oder verschiedenen Halogenatomen;

$R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, geradkettiges oder verzweigtes Halogenalkyl mit 1 bis 4 Kohlensloffatomen und 1 bis 9 gleichen oder verschiedenen Halogenatomen, insbesondere Fluor, Chlor oder Brom, für Methoxymethyl, Ethoxymethyl, Propoxymethyl, Cyclopentyl, Cyclohexyl, Cyclopropyl, Cyclopropylmethyl, Cyclohexylmethyl, Cyclohexylethyl, für Tetrahydrofuranyl, für Tetrahydrofuranylmethyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;

$R^2$ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen:

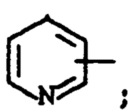 ; 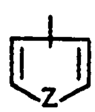 ; 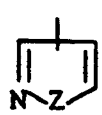 ; 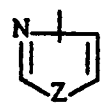 ;

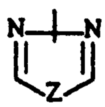 ; 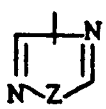 ; 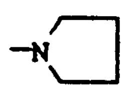 ; 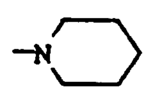 ;

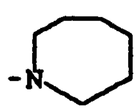 ; 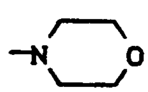 oder 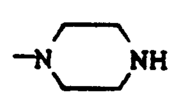

wobei Z jeweils für Sauerstoff oder Schwefel steht und wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^2$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls

geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy,

$R^3$ für Methyl, Ethyl, n-Propyl, n-Butyl, i-Propyl, i-Butyl oder Cyclopropyl steht,

X für Sauerstoff oder Schwefel steht und

Y für Sauerstoff oder Schwefel steht.

Ganz besonders bevorzugt sind Verbindungen der Formel (I), bei welchen

$R^1$ für Wasserstoff, Methyl, Ethyl, n- oder i-Propyl, Methoxymethyl, Ethoxymethyl oder Propoxymethyl steht,

$R^2$ für Wasserstoff, für jeweils gegebenenfalls ein-bis dreifach durch Fluor und/oder Chlor substituiertes Methyl, Ethyl, n- oder i-Propyl, n-, i-, s- oder t-Butyl, Allyl, Propargyl, jeweils geradkettiges oder verzweigtes Pentyl, Hexyl, Heptyl, Octyl, Butenyl, Pentenyl, Hexenyl, Butinyl, Pentinyl oder Hexinyl steht; außerdem für jeweils gegebenenfalls ein- bis dreifach gleich oder verschieden durch Fluor, Chlor, Methyl, Ethyl und/oder Cyano substituiertes Cyclopropyl, Cyclopentyl, Cyclohexyl, Cyclohexenyl, Cyclopropylmethyl, Cyclopropylethyl, Cyclohexylmethyl, Cyclohexylethyl oder Cycloheptyl steht und schließlich für Benzyl, Phenylethyl oder Phenyl steht,

$R^3$ für Methyl, Ethyl, n-Propyl oder Isopropyl steht,

X für Sauerstoff oder Schwefel steht und

Y für Sauerstoff oder Schwefel steht.

Im einzelnen seien außer den bei den Herstellungsbeispielen genannten Verbindungen die folgenden substituierten Triazolinone der allgemeinen Formel (I) genannt:

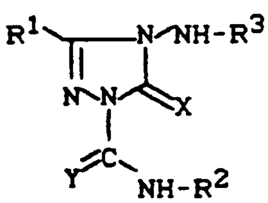

(I)

| $R^1$ | $R^2$ | $R^3$ | X | Y |
|---|---|---|---|---|
| $CH_3$ | 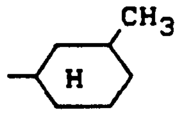 | $CH_3$ | O | O |
| $CH_3$ | 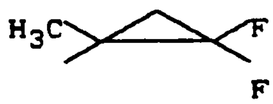 | $CH_3$ | O | O |
| $CH_3$ | 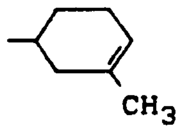 | $CH_3$ | O | O |
| $CH_3$ | $-C(CH_3)_3$ | $CH_3$ | O | S |
| $CH_3$ | $-C(CH_3)_3$ | $CH_3$ | S | S |
| $CH_3$ | 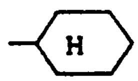 | $CH_3$ | S | S |
| $CH_3$ | 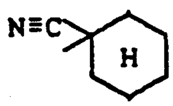 | $CH_3$ | O | O |
| $C_2H_5$ | $-C(CH_3)_3$ | $CH_3$ | O | O |
| $C_2H_5$ | $-C(CH_3)_3$ | $CH_3$ | O | S |
| $C_2H_5$ | $-C(CH_3)_3$ | $CH_3$ | S | O |

| $R^1$ | $R^2$ | $R^3$ | X | Y |
|---|---|---|---|---|
| $C_2H_5$ | 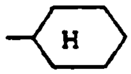 | $CH_3$ | O | S |
| $C_2H_5$ | 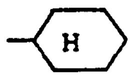 | $CH_3$ | S | O |
| $C_2H_5$ | 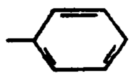 | $C_2H_5$ | O | O |
| $C_2H_5$ | 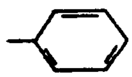 | $C_2H_5$ | O | S |
| $C_2H_5$ | 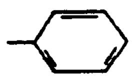 | $C_2H_5$ | S | O |
| $C_2H_5$ | 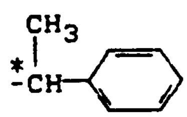 S-Konfiguration | $CH_3$ | O | O |
| H | $-C(CH_3)_3$ | $C_3H_7$-i | O | O |
| H | 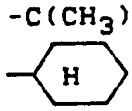 | $CH_3$ | O | O |
| H | 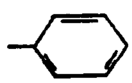 | $C_3H_7$ | O | O |
| H | $-C(CH_3)_3$ | $CH_3$ | O | S |
| H | $-C(CH_3)_3$ | $CH_3$ | S | O |
| H | 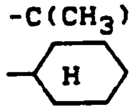 | $CH_3$ | O | S |
| H | 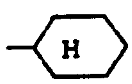 | $CH_3$ | S | O |
| $CH_3$ | 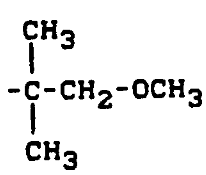 | $CH_3$ | O | O |

| $R^1$ | $R^2$ | $R^3$ | X | Y |
|---|---|---|---|---|
| $CH_3$ | 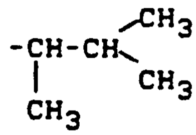 | $CH_3$ | O | O |
| $CH_3$ | 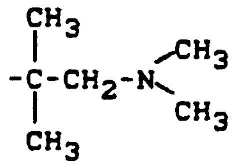 | $CH_3$ | O | O |
| $CH_3$ | 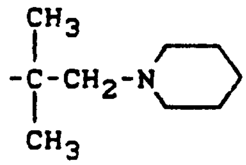 | $CH_3$ | O | O |
| $CH_3$ | 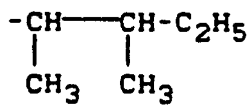 | $CH_3$ | O | O |
| $CH_3$ | 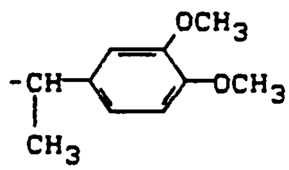 | $CH_3$ | O | O |
| $CH_3$ | 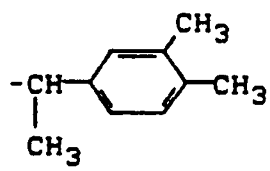 | $CH_3$ | O | O |
| $CH_3$ | 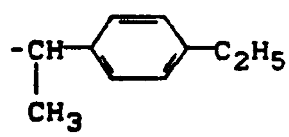 | $CH_3$ | O | O |
| $CH_3$ | 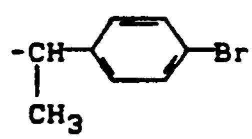 | $CH_3$ | O | O |

| $R^1$ | $R^2$ | $R^3$ | X | Y |
|---|---|---|---|---|
| $CH_3$ | 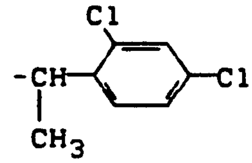 | $C_2H_5$ | O | O |
| $CH_3$ | 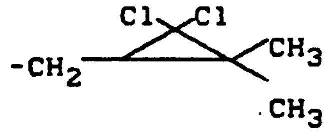 | $CH_3$ | O | O |
| $CH_3$ | 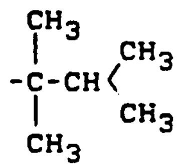 | $CH_3$ | O | O |
| $CH_3$ | 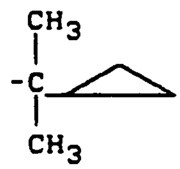 | $C_2H_5$ | O | O |
| $CH_3$ | 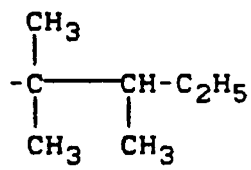 | $CH_3$ | O | O |
| $CH_3$ | 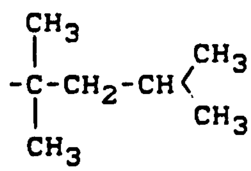 | $CH_3$ | O | O |
| $CH_3$ | 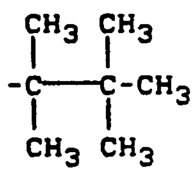 | $CH_3$ | O | O |

| $R^1$ | $R^2$ | $R^3$ | X | Y |
|---|---|---|---|---|
| $CH_3$ | 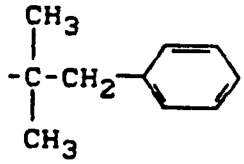 | $CH_3$ | O | O |
| $CH_3$ | 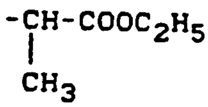 | $CH_3$ | O | O |
| $CH_3$ | 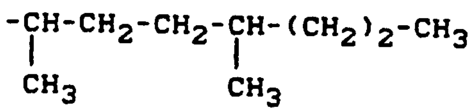 | $CH_3$ | O | O |
| $CH_3$ | 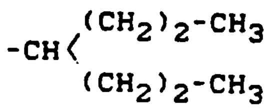 | $CH_3$ | O | O |
| $CH_3$ | 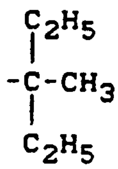 | $CH_3$ | O | O |
| $CH_3$ | 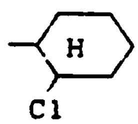 | $CH_3$ | O | O |
| $CH_3$ | 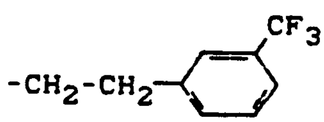 | $CH_3$ | O | O |
| $CH_3$ | 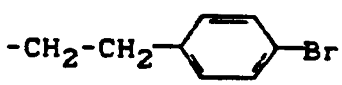 | $C_2H_5$ | O | O |
| $CH_3$ | 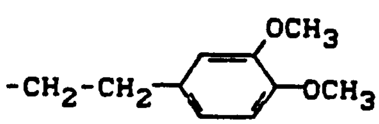 | $C_2H_5$ | O | O |

| $R^1$ | $R^2$ | $R^3$ | X | Y |
|---|---|---|---|---|
| $CH_3$ | 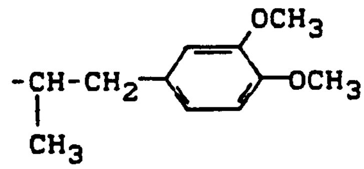 | $C_2H_5$ | O | O |
| $CH_3$ | 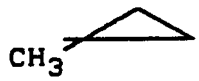 | 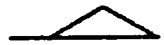 | O | O |
| $CH_3$ | 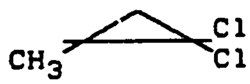 | $CH_3$ | O | O |
| $CH_3$ | 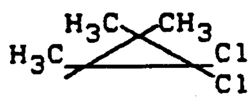 | $CH_3$ | O | O |
| $CH_3$ | 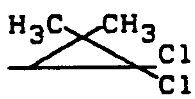 | $CH_3$ | O | O |
| $CH_3$ | 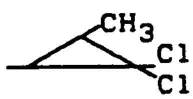 | $CH_3$ | O | O |
| $CH_3$ | 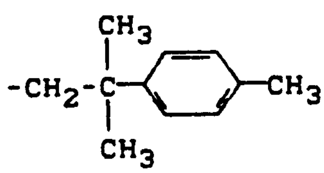 | $CH_3$ | O | O |
| $CH_3$ | 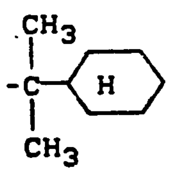 | $CH_3$ | O | O |
| $CH_3$ | 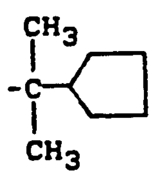 | $CH_3$ | O | O |

| $R^1$ | $R^2$ | $R^3$ | X | Y |
|---|---|---|---|---|
| $CH_3$ | 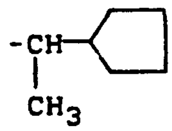 | $CH_3$ | O | O |
| $CH_3$ | 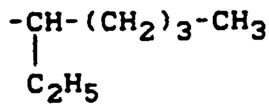 | $CH_3$ | O | O |
| $CH_3$ | 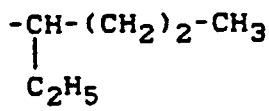 | $CH_3$ | O | O |
| $CH_3$ | 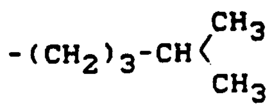 | $CH_3$ | O | O |
| $CH_3$ | 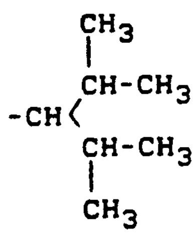 | $CH_3$ | O | O |
| $CH_3$ | 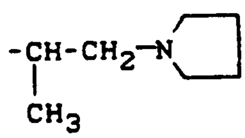 | $CH_3$ | O | O |
| $CH_3$ | 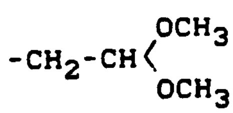 | $CH_3$ | O | O |
| $CH_3$ | 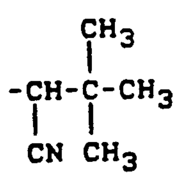 | $CH_3$ | O | O |
| $CH_3$ | 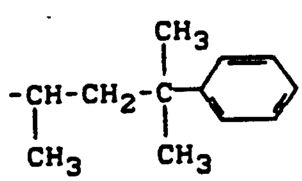 | $CH_3$ | O | O |

| $R^1$ | $R^2$ | $R^3$ | X | Y |
|---|---|---|---|---|
| $CH_3$ | 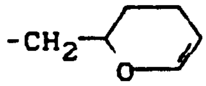 | $CH_3$ | O | O |
| $CH_3$ | 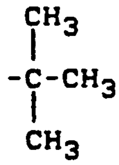 | $CH_3$ | O | S |
| $CH_3$ | 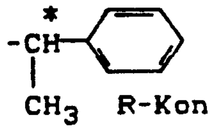 R-Konfiguration | $CH_3$ | O | S |
| $CH_3$ | 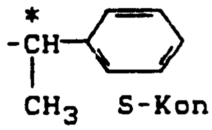 S-Konfiguration | $CH_3$ | O | S |
| $CH_3$ | 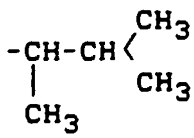 | $CH_3$ | O | S |
| $CH_3$ | 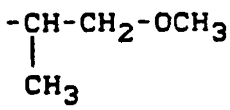 | $CH_3$ | O | S |
| $CH_3$ | $-(CH_2)_2-CH_3$ | $CH_3$ | O | S |
| $CH_3$ | 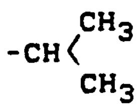 | $CH_3$ | O | S |
| $CH_3$ | 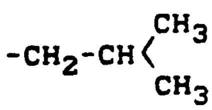 | $CH_3$ | O | S |
| $CH_3$ | 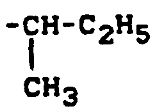 | $CH_3$ | O | S |

| $R^1$ | $R^2$ | $R^3$ | X | Y |
|---|---|---|---|---|
| $CH_3$ | 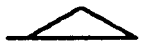 | $CH_3$ | O | S |
| $CH_3$ | 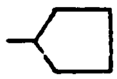 | $CH_3$ | O | S |
| $CH_3$ | $-(CH_2)_2-CH_3$ | $CH_3$ | S | O |
| $CH_3$ | $-(CH_2)_3-CH_3$ | $CH_3$ | S | O |
| $CH_3$ | 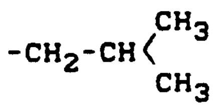 | $CH_3$ | S | O |
| $CH_3$ | 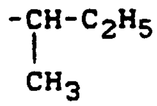 | $CH_3$ | S | O |

Verwendet man beispielsweise n-Butylisocyanat und 3-Methyl-4-methylamino-(1H)-1,2,4-triazolin-5-on als Ausgangsverbindungen, so läßt sich der Reaktionsablauf des erfindungsgemäßen Verfahrens (a) durch das folgende Formelschema darstellen:

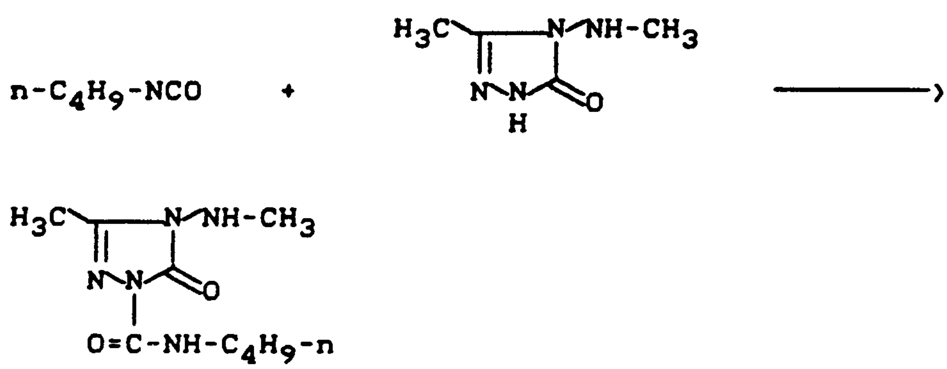

Verwendet man beispielsweise α,α-Dimethylbenzylisocyanat und 3-Isopropyl-4-ethoxymethylenimino-1H-1,2,4-triazolin-5-on als Ausgangsverbindungen, so läßt sich der Reaktionsablauf des erfindungsgemäßen Verfahrens (b) durch das folgende Formelschema darstellen:

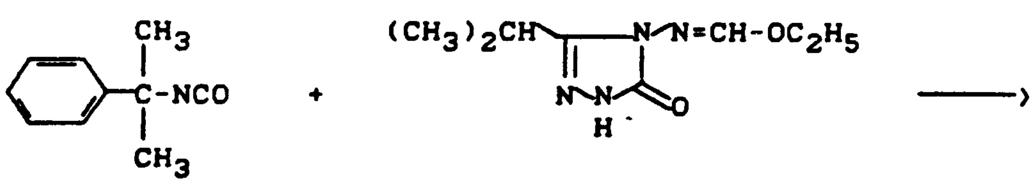

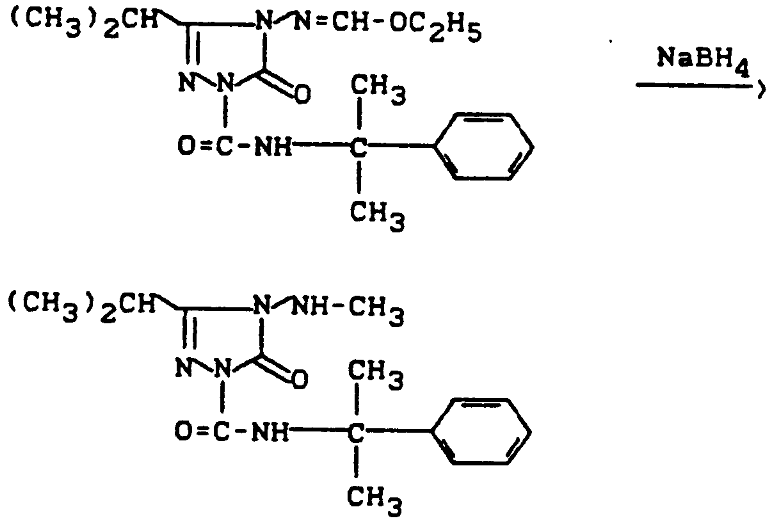


Verwendet man beispielsweise 1-(N-Phenylcarbamoyl)-3-trifluormethyl-4-amino-1,2,4-triazolin-5-on und Triethyl-orthoacetat als Ausgangsstoffe, so läßt sich der Reaktionsablauf des erfindungsgemäßen Verfahrens (c) durch das folgende Formelschema darstellen:

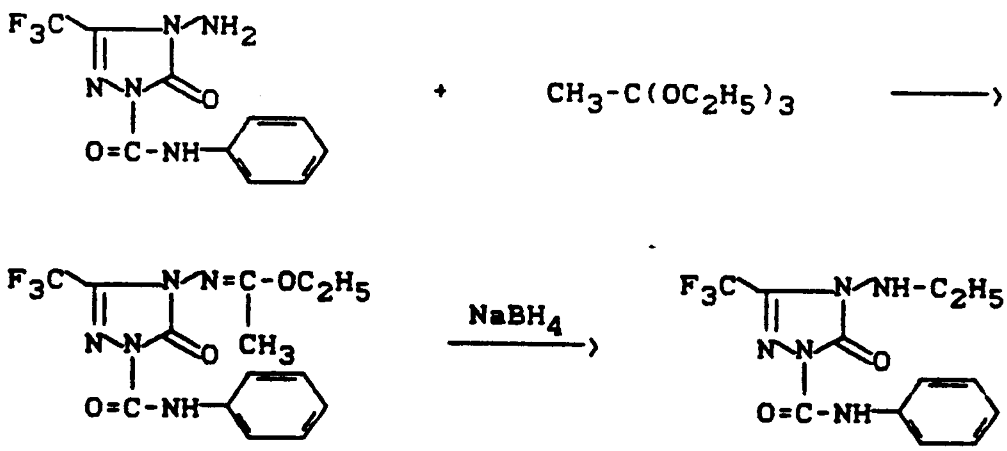


Verwendet man beispielsweise 3-Methyl-4-phenylamino-1H-1,2,4-triazolin-5-on, Chlorthioameisensäure-O-phenylester und Cyclopentylamin als Ausgangsstoffe, so läßt sich der Reaktionsablauf des erfindungsgemäßen Verfahrens (d) durch das folgende Formelschema darstellen:

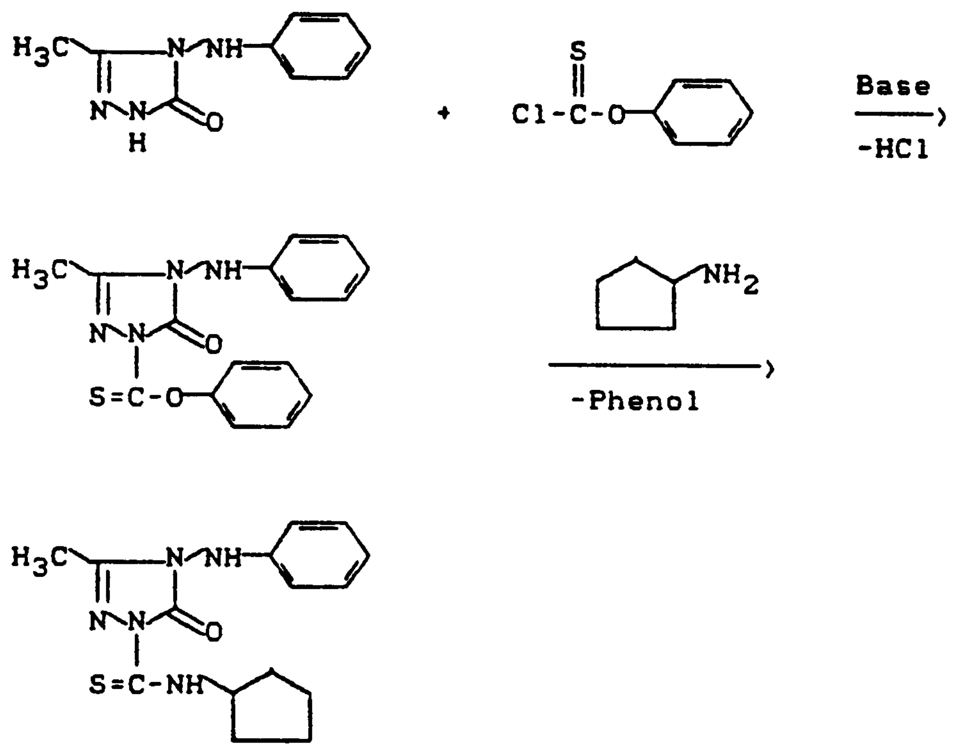


Verwendet man beispielsweise N-Benzyl-O-phenyl-urethan und 3-Cyclobutyl-4-isopropylamino-1H-1,2,4-triazolin-5-on als Ausgangsstoffe, so läßt sich der Reaktionsablauf des erfindungsgemäßen Verfahrens (e) durch das folgende Formelschema darstellen:

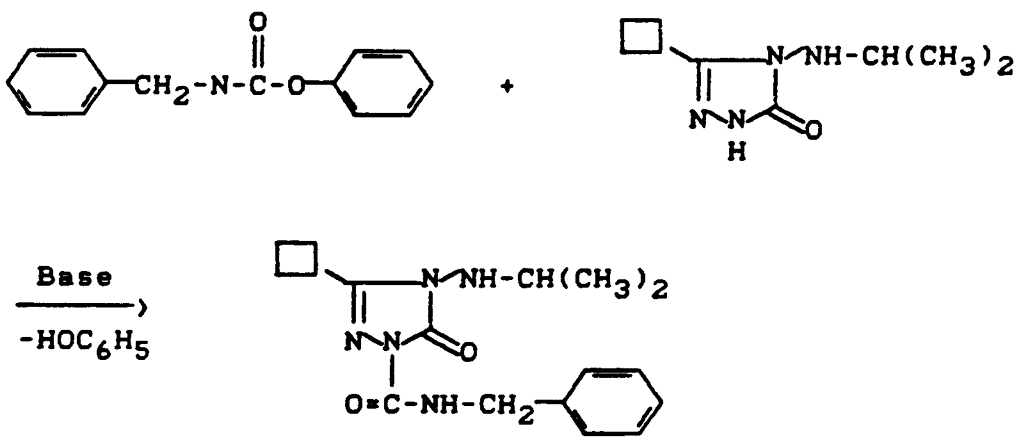


Verwendet man beispielsweise 1-Cyclopropylaminocarbonyl-3-(1,1-dimethyl-ethyl)-4-benzylidenimino-1,2,4-triazolin-5-on und Natrium-cyanoborhydrid als Ausgangsstoffe, so läßt sich der Reaktionsablauf des erfindungsgemäßen Verfahrens (f) durch das folgende Formelschema darstellen:

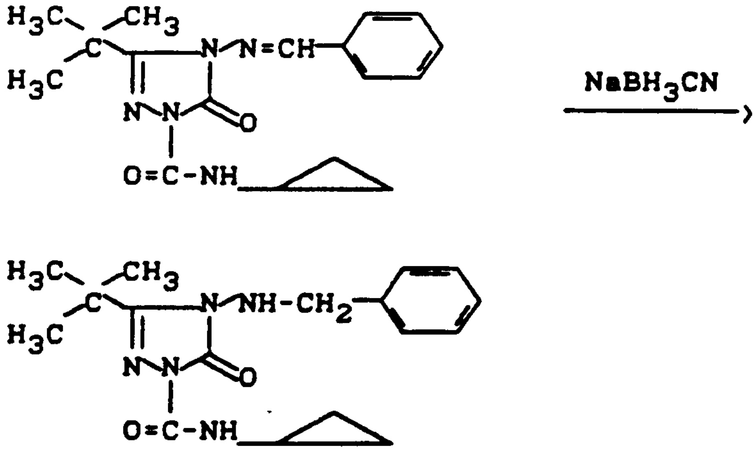


Die beim erfindungsgemäßen Verfahren (a) zur Herstellung von Verbindungen der Formel (I) als Ausgangsstoffe zu verwendenden 1H-Triazolinone sind durch die Formel (II) allgemein definiert.

In Formel (II) haben $R^1$, $R^3$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^3$ und X angegeben wurden.

1H-Triazolin(thi)one der Formel (II) sind teilweise aus der Literatur bekannt. (Siehe hierzu GB-A-2 194 534; DE-A-2 416 814; FR-A-2 546 887; Chem. Abstr. 103: 22 524 w; Chem. Abstr. 80: 27 172 w; Chem. Abstr. 94: 15 645 d; Chem. Abstr. 83: 205 712 u; Chem. Abstr. 95: 115 397 p; Chem. Abstr. 76: 85 755 s; Chem. Abstr. 110: 222 506 y; Chem. Abstr. 97: 6 227 d; Chem. Abstr. 88: 120169 a;)
Man erhält die neuen Verbindungen der Formel (II), wenn man Hydrazide der allgemeinen Formel (XVI)

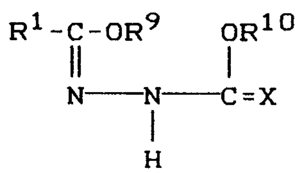
(XVI)

in welcher

$R^1$ und X die oben angegebene Bedeutung haben und

$R^9$ und $R^{10}$ unabhängig voneinander für Alkyl, Aryl oder Aralkyl stehen,

mit Hydrazinderivaten der allgemeinen Formel (XVII)

$H_2N\text{-}NH\text{-}R^3$ (XVII)

in welcher

$R^3$ die oben angegebene Bedeutung hat,

gegebenenfalls in Gegenwart eines Verdünnungsmittels, wie z.B. Dichlormethan, Chloroform, Toluol, Chlorbenzol, Mesitylen oder Ethylenglycoldiethylether, und gegebenenfalls in Gegenwart eines Katalysators, wie z.B. 1,8-Diazabicyclo[5,4,0]-undec-7-en (DBU), Triethylamin oder Kaliumcarbonat, bei Temperaturen zwischen 50 °C und 200 °C umsetzt.

Die Ausgangsstoffe der Formeln (XVI) und (XVII) sind bekannt (vgl. Bull. Soc. Chim. France 1962, 1364; Chim. Acta Turcica 3 (1975), 113).

Die bei den erfindungsgemäßen Verfahren (a) und (b) als Ausgangsstoffe zu verwendenden Iso(thio)-cyanate sind durch die Formel (III) allgemein definiert.

In Formel (III) haben $R^2$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und Y angegeben wurden.

Die Iso(thio)cyanate der Formel (III) sind bekannte organische Synthesechemikalien.

Die beim erfindungsgemäßen Verfahren (b) als Ausgangsstoffe zu verwendenden 1H-Triazolinone sind durch die Formel (IV) allgemein definiert.

In Formel (IV) haben $R^1$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und X angegeben wurden;

$R^4$ steht vorzugsweise für Wasserstoff, $C_1$-$C_4$-Alkyl, $C_2$-$C_4$-Alkenyl, $C_2$-$C_4$-Alkinyl, $C_3$-$C_6$-Cycloalkyl, Phenyl oder Benzyl, und

$R^5$ steht vorzugsweise für $C_1$-$C_4$-Alkyl, $C_2$-$C_4$-Alkenyl, $C_2$-$C_4$-Alkinyl, $C_3$-$C_6$-Cycloalkyl, Phenyl oder Benzyl.

Die 1H-Triazolinone der Formel (IV) sind bekannt und/oder können nach an sich bekannten Verfahren hergestellt werden (vgl. J. Heterocycl. Chem. 17 (1980), 1691-1696).

Man erhält die Verbindungen der Formel (IV), wenn man Aminotriazolinone der allgemeinen Formel (XVIII)

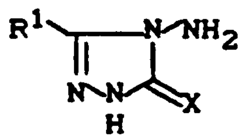

(XVIII)

in welcher

$R^1$ und X die oben angegebene Bedeutung haben,

mit Orthocarbonsäureestern der allgemeinen Formel (X)

$R^4$-$C(OR^5)_3$ (X)

in welcher

$R^4$ und $R^5$ die oben angegebene Bedeutung haben,

gegebenenfalls in Gegenwart eines Verdünnungsmittels, wie z.B. Chloroform, Toluol oder Chlorbenzol, und gegebenenfalls in Gegenwart eines Katalysators, wie z.B. p-Toluolsulfonsäure, bei Temperaturen zwischen 50 °C und 200 °C umsetzt.

Die Ausgangsstoffe der Formeln (X) und (XVIII) sind bekannt (vgl. Chem. Ber. 98 (1965), 3025-3033; Chimica Acta Turcica 7 (1979), 269; J. Heterocycl. Chem. 16 (1979), 403-407).

Die beim erfindungsgemäßen Verfahren (b) als Intermediate isolierbaren Verbindungen der Formel (V) sind noch nicht aus der Literatur bekannt. Die neuen Verbindungen der Formel (V) werden als erfindungsgemäße neue Stoffe beansprucht.

In Formel (V) haben $R^1$, $R^2$, $R^4$, $R^5$, X und Y vorzugsweise bzw. insbesondere die bereits oben für $R^1$, $R^2$, $R^4$, $R^5$, X und Y als bevorzugt bzw. als insbesondere bevorzugt angegebene Bedeutung.

Die in der zweiten Stufe des erfindungsgemäßen Verfahrens (b) zu verwendenden Reduktionsmittel sind durch die Formeln (VI) und (VII) allgemein definiert. In den Formeln (VI) und (VII) steht M jeweils vorzugsweise für Lithium, Natrium oder Kalium, insbesondere für Lithium oder Natrium.

Die Reduktionsmittel der Formeln (VI) und (VII) sind bekannte Synthesechemikalien.

Die nach Verfahren (b) herzustellenden Verbindungen der Formel (VIII) stellen eine Auswahl aus den erfindungsgemäßen Verbindungen der Formel (I) dar und sind somit ebenfalls erfindungsgemäße neue Stoffe.

Die beim erfindungsgemäßen Verfahren (c) zur Herstellung von Verbindungen der Formel (I) als Ausgangsstoffe zu verwendenden 1-Carbamoyl-triazolinone sind durch die Formel (IX) allgemein definiert.

In Formel (IX) haben $R^1$, $R^2$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, X und Y angegeben wurden.

Die Ausgangsstoffe der Formel (IX) sind bekannt und/oder können nach an sich bekannten Verfahren hergestellt werden (vgl. EP-A 294666).

Bezüglich der bei Verfahren (c) weiter als Ausgangsstoffe zu verwendenden Verbindungen der Formeln (VI), (VII) und (X) bzw. der als Produkte anfallenden Verbindungen der Formeln (V) und (VIII) gelten die gleichen Angaben, die bei der Beschreibung des erfindungsgemäßen Verfahrens (b) gemacht wurden.

Bezüglich der beim erfindungsgemäßen Verfahren (d) als Ausgangsstoffe zu verwendenden 1H-Triazolinone der Formel (II) gelten die oben bei der Beschreibung der Ausgangsstoffe für das erfindungsgemäße Verfahren (a) gemachten Angaben.

Die beim erfindungsgemäßen Verfahren (d) zur Herstellung von Verbindungen der Formel (I) weiter als Ausgangsstoffe zu verwendenden (Thio)Chlorameisensäureester sind durch die Formel (XI) allgemein definiert.

In Formel (XI) hat Y vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Y angegeben wurde und $R^6$ steht vorzugsweise für $C_1$-$C_4$-Alkyl, Phenyl oder Benzyl.

Die Ausgangsstoffe der Formel (XI) sind bekannte organische Synthesechemikalien.

Die bei der zweiten Stufe des erfindungsgemäßen Verfahrens (d) als Ausgangsstoffe zu verwendenden Amine sind durch die Formel (XIII) allgemein definiert.

In Formel (XIII) hat $R^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^2$ angegeben wurde.

Die Ausgangsstoffe der Formel (XIII) sind bekannte Synthesechemikalien.

Die beim erfindungsgemäßen Verfahren (d) als Intermediate isolierbaren Verbindungen der Formel (XII) sind noch nicht aus der Literatur bekannt. Die neuen Verbindungen der Formel (XII) werden als erfindungsgemäße neue Stoffe beansprucht.

In Formel (XII) haben $R^1$, $R^3$, $R^6$, X und Y vorzugsweise bzw. insbesondere die bereits oben für $R^1$, $R^3$, $R^6$, X und Y als bevorzugt bzw. als insbesondere bevorzugt angegebene Bedeutung.

Die bei den erfindungsgemäßen Verfahren (b) und (c) anfallenden Intermediate der Formel (V) und die bei Verfahren (d) erhaltenen Intermediate der Formel (XII) können jeweils isoliert werden, aber auch ohne Zwischenisolierung (im sog. "Eintopf-Verfahren") weiter umgesetzt werden.

Bezüglich der beim erfindungsgemäßen Verfahren (e) als Ausgangsstoffe zu verwendenden 1H-Triazolinone der Formel (II) gelten die oben bei der Beschreibung der Ausgangsstoffe für das erfindungsgemäße Verfahren (a) gemachten Angaben.

Die beim erfindungsgemäßen Verfahren (e) zur Herstellung von Verbindungen der Formel (I) weiter als Ausgangsstoffe zu verwendenden (Thio)Urethane sind durch die Formel (XIV) allgemein definiert.

In Formel (XIV) haben $R^2$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und Y angegeben wurden, und $R^6$ steht vorzugsweise für $C_1$-$C_4$-Alkyl, Phenyl oder Benzyl.

Die Ausgangsstoffe der Formel (XIV) sind bekannte organische Synthesechemikalien.

Die beim erfindungsgemäßen Verfahren (f) zur Herstellung von Verbindungen der Formel (I) als Ausgangsstoffe zu verwendenden Triazolinon-hydrazone sind durch die Formel (XV) allgemein definiert.

In Formel (XV) haben $R^1$, $R^2$, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, X und Y angegeben wurden und $R^7$ und $R^8$ stehen vorzugsweise jeweils unabhängig voneinander für Wasserstoff, $C_1$-$C_4$-Alkyl, Phenyl oder Benzyl.

Die Ausgangsstoffe der Formel (XV) sind bekannt und/oder können nach an sich bekannten Verfahren hergestellt werden (vgl. EP-A 294666).

Das erfindungsgemäße Verfahren (a) wird vorzugsweise in Gegenwart eines Verdünnungsmittels durchgeführt.

Als Verdünnungsmittel zur Durchführung des erfindungsgemäßen Verfahrens (a) kommen insbesondere inerte organische Lösungsmittel infrage. Hierzu gehören beispielsweise aliphatische, alicyclische oder aromatische, gegebenenfalls halogenierte Kohlenwasserstoffe, wie Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (a) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man pro Mol an 1H-Triazolinon der Formel (II) im allgemeinen 1.0 bis 2.0 Mol, vorzugsweise 1.0 bis 1.5 Mol an Iso(thio)cyanat der Formel (III) und gegebenenfalls 0.001 bis 2.0 Mol, vorzugsweise 0.001 bis 1.0 Mol an Reaktionshilfsmittel ein.
Die Reaktionsdurchführung, Aufarbeitung und Isolierung der Reaktionsprodukte erfolgt nach allgemein üblichen Methoden.

Die erste Stufe des erfindungsgemäßen Verfahrens (b) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Auch bezüglich der Verwendung eines Reaktionshilfsmittels und der Reaktionstemperaturen gelten bei Verfahren (b) in der ersten Stufe die oben für Verfahren (a) gemachten Angaben.

Zur Durchführung des erfindungsgemäßen Verfahrens (b) setzt man auf 1 Mol 1H-Triazolinon der Formel (IV) im allgemeinen 1,0 bis 2,0 Mol, vorzugsweise 1,0 bis 1,5 Mol, Iso(thio)cyanat der Formel (III) und gegebenenfalls 0,001 bis 2,0 Mol, vorzugsweise 0,01 bis 1,0 Mol, Reaktionshilfsmittel ein.

Die Reaktionsdurchführung und gegebenenfalls die Aufarbeitung und Isolierung der Intermediate der Formel (V) kann nach allgemein üblichen Methoden erfolgen.

Die zweite Stufe des erfindungsgemäßen Verfahrens (b) wird vorzugsweise in Gegenwart eines polaren Lösungsmittels durchgeführt. Als solche kommen vorzugsweise Wasser, Alkohole wie Methanol, Ethanol, Propanol, Isopropanol, Butanol, Isobutanol, sec-Butanol und tert-Butanol, Etheralkohole wie Methoxyethanol und Ethoxyethanol, oder Ether wie Diethylether, Dipropylether, Diisopropylether, Tetrahydrofuran und Dioxan, in Betracht.

Die Reaktionstemperaturen können bei der zweiten Stufe von Verfahren (b) in einem größeren Bereich variiert werden. Im allgemeinen arbeitet man bei Temperaturen zwischen -20 °C und +100 °C, vorzugsweise zwischen 0 °C und +30 °C.

Zur Durchführung der zweiten Stufe von Verfahren (b) setzt man auf 1 Mol Intermediat der Formel (V) im allgemeinen 0,5 bis 5 Mol, vorzugsweise 1 bis 3 Mol, Reduktionsmittel der Formel (VI) oder (VII) ein.

Die Reaktionsdurchführung, Aufarbeitung und Isolierung der Reaktionsprodukte erfolgt nach allgemein üblichen Methoden.

Die erste Stufe des erfindungsgemäßen Verfahrens (c) wird gegebenenfalls in Gegenwart eines Verdünnungsmittels durchgeführt. Es können die gleichen Lösungsmittel verwendet werden, die oben für das erfindungsgemäße Verfahren (a) angegeben sind.

Verfahren (c) wird in der ersten Stufe vorzugsweise in Gegenwart eines Reaktionshilfsmittels durchgeführt. Als solche sind hierbei insbesondere starke Säuren, wie z.B. Hydrogenchlorid, Schwefelsäure, Methansulfonsäure, Benzolsulfonsäure und p-Toluolsulfonsäure, geeignet.

Die Reaktionstemperaturen können in der ersten Stufe des erfindungsgemäßen Verfahrens (c) in einem größeren Bereich variiert werden. Im allgemeinen arbeitet man bei Temperaturen zwischen 0 °C und 200 °C, vorzugsweise bei Temperaturen zwischen 50 °C und 150 °C.

Zur Durchführung des erfindungsgemäßen Verfahrens (c) setzt man auf 1 Mol 1-Carbamoyl-triazolinon der Formel (IX) im allgemeinen 1 bis 100 Mol, vorzugsweise 1 bis 20 Mol, Orthocarbonsäureester der Formel (X) und gegebenenfalls 0,001 bis 2,0 Mol, vorzugsweise 0,01 bis 1,0 Mol, Reaktionshilfsmittel ein.
Die Reaktionsdurchführung und gegebenenfalls die Aufarbeitung und Isolierung der Intermediate der Formel (V) kann nach allgemein üblichen Methoden erfolgen.

Die zweite Stufe des erfindungsgemäßen Verfahrens (c) wird vorzugsweise in Gegenwart eines Verdünnungsmittels durchgeführt. Es können hierbei die gleichen Lösungsmittel verwendet werden, die oben für die zweite Stufe von Verfahren (b) angegeben sind.

Die Reaktionstemperaturen können bei der zweiten Stufe von Verfahren (c) in einem größeren Bereich variiert werden. Im allgemeinen arbeitet man bei Temperaturen zwischen -20 °C und +100 °C, vorzugsweise zwischen 0 °C und +30 °C.

Zur Durchführung von Verfahren (c) setzt man in der zweiten Stufe auf 1 Mol Intermediat der Formel (V) im allgemeinen 0,5 bis 5 Mol, vorzugsweise 1 bis 3 Mol, Reduktionsmittel der Formel (VI) oder (VII) ein.

Die Reaktionsdurchführung, Aufarbeitung und Isolierung der Reaktionsprodukte erfolgt nach üblichen Methoden.

Das erfindungsgemäße Verfahren (d) wird in beiden Stufen vorzugsweise in Gegenwart eines Verdünnungsmittels durchgeführt. Es können hierbei die gleichen Lösungsmittel verwendet werden, die oben für das erfindungsgemaße Verfahren (a) angegeben sind.

Das erfindungsgemäße Verfahren (d) kann in beiden Stufen gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der beiden Stufen des erfindungsgemäßen Verfahrens (d) in einem größeren Bereich variiert werden. Im allgemeinen arbeitet man bei Temperaturen zwischen 0 °C und 150 °C, vorzugsweise zwischen 10 °C und 100 °C.

Zur Durchführung des erfindungsgemäßen Verfahrens (d) setzt man auf 1 Mol 1H-Triazolinon der Formel (II) im allgemeinen 1 bis 2 Mol, vorzugsweise 1,0 bis 1,5 Mol, (Thio)Chlorameisensäureester der Formel (XI) und 1 bis 2 Mol, vorzugsweise 1,0 bis 1,5 Mol, Reaktionshilfsmittel ein.

Die Reaktionsdurchführung und gegebenenfalls die Aufarbeitung und Isolierung der Intermediate der Formel (XII) kann nach allgemein üblichen Methoden erfolgen.

Zur Durchführung der zweiten Stufe von Verfahren (d) setzt man auf 1 Mol Intermediat der Formel (XII) im allgemeinen 1 bis 2 Mol, vorzugsweise 1,0 bis 1,5 Mol, Amin der Formel (XIII) ein.

Die Reaktionsdurchführung, Aufarbeitung und Isolierung der Produkte der Formel (I) erfolgt nach allgemein üblichen Methoden.

Das erfindungsgemäße Verfahren (e) wird vorzugsweise in Gegenwart eines Verdünnungsmittels durchgeführt. Es können hierbei die gleichen Lösungsmittel verwendet werden, die oben für das erfindungsgemäße Verfahren (a) angegeben sind.

Verfahren (e) wird vorzugsweise in Gegenwart eines Reaktionshilfsmittels durchgeführt. Es können hierbei die gleichen Reaktionshilfsmittel eingesetzt werden, die oben für das erfindungsgemäße Verfahren (d) angegeben sind.

Die Reaktionstemperaturen können bei dem erfindungsgemäßen Verfahren (e) in einem größeren Bereich variiert werden. Im allgemeinen arbeitet man bei Temperaturen zwischen 0 °C und 150 °C, vorzugsweise bei Temperaturen zwischen 10 °C und 120 °C.

Zur Durchführung des erfindungsgemäßen Verfahrens (e) setzt man auf 1 Mol 1H-Triazolinon der Formel (II) im allgemeinen 1 bis 2 Mol, vorzugsweise 1,0 bis 1,5 Mol, (Thio)Urethan der Formel (XIV) ein.

Die Reaktionsdurchführung, Aufarbeitung und Isolierung der Produkte der Formel (I) erfolgt nach allgemein üblichen Methoden.

Das erfindungsgemäße Verfahren (f) wird unter Verwendung eines Reduktionsmittels und gegebenenfalls eines Katalysators durchgeführt. Geeignete Systeme aus Reduktionsmitteln und Katalysatoren sind beispielsweise Wasserstoff in Kombination mit üblichen Hydrierungskatalysatoren, wie z.B. Raney-Nickel, Palladium oder Platin, ferner auch gegebenenfalls komplexe Metallhydride, wie z.B. Lithiumalanat, Natriumboranat und Natriumcyanoborhydrid, gegebenenfalls in Kombination mit sauren Katalysatoren, wie z.B. Salzsäure oder Essigsäure.

Verfahren (f) wird vorzugsweise in Gegenwart eines Verdünnungsmittels durchgeführt. Es können hierbei die gleichen Lösungsmittel verwendet werden, die oben für die zweite Stufe des erfindungsgemäßen Verfahrens (b) angegeben sind.

Die Reaktionstemperaturen können bei dem erfindungsgemäßen Verfahren (f) in einem größeren Bereich variiert werden. Im allgemeinen arbeitet man bei Temperaturen zwischen -20 °C und +100 °C, vorzugsweise bei Temperaturen zwischen 0 °C und +30 °C.

Die Reaktionsdurchführung, Aufarbeitung und Isolierung der Reaktionsprodukte erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattunagen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, sowohl im Vorauflauf- als auch im Nachauflauf-Verfahren.

Die erfindungsgemäßen Wirkstoffe weisen ferner eine starke Wirkung gegen Schädlinge auf und können zur Bekämpfung unerwünschter Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum; Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Bei der Verwendung der Wirkstoffe als Fungizide sind ferner auch zusätzlich Feinstverkapselungen in Hüllmassen für Saatgut, sowie ULV-Kalt- und Warmnebelformulierungen einsetzbar.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage, ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methyl-benzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin(TERBUTRYNE); 3-[[[[(4-Methoxy-6-methyl-1,3,5-triazin-2-yl)-amino]carbonyl]amino]-sulfonyl]-thiophen-2-carbonsäure-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich. Bei der Anwendung als Fungizid sind auch Mischungen mit Düngemitteln und Wachstumsregulatoren möglich.

Die Wirkstoffe können - je nach Anwendung - als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die erfindungsgemäßen Wirkstoffe können bei der Anwendung als Herbizid sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizid in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

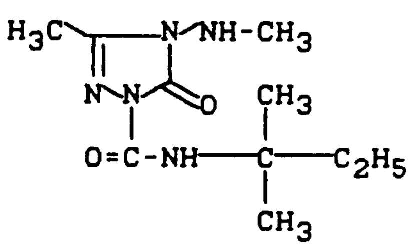

(Verfahren (a))

3,9 g (0,03 Mol) 3-Methyl-4-methylamino-1H-1,2,4-triazolin-5-on werden in 80 ml Acetonitril aufgeschlämmt und nacheinander mit 0,2 g Diazabicycloundecen (DBU) und 3,7 g (0,033 Mol) tert-Amylisocyanat versetzt. Man rührt 12 Stunden bei 20 °C, engt im Vakuum ein, nimmt in Methylenchlorid auf und wäscht mit verdünnter Salzsäure und Wasser neutral. Die organische Phase wird getrocknet, eingeengt und der Rückstand mit Ether/Petrolether verrieben.

Man erhält 5.0 g (0,0207 Mol, 69% der Theorie) an 1-[N-(1,1-Dimethyl-propyl)-carbamoyl]-3-methyl-4-methylamino-1,2,4-triazolin-5-on vom Schmelzpunkt 98 °C.

Beispiel 2

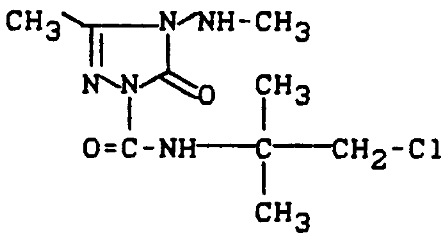

(Verfahren (b))

8,5 g (0,05 Mol) 4-Ethoxymethylenimino-3-methyl-1H-1,2,4-triazolin-5-on werden in 100 ml Acetonitril aufgeschlämmt und unter Rühren nacheinander mit ca. 200 mg Diazabicycloundecen (DBU) und 6,7 g (0,05 Mol) Monochlor-tert-butylisocyanat versetzt. Nach 12 Stunden bei 20 °C wird im Vakuum eingeengt, in Methylenchlorid aufgenommen und mit verdünnter Salzsäure und Wasser neutral gewaschen. Nach dem Trocknen wird eingeengt und der Rückstand mit n-Hexan verrieben.

Man erhält 11,0 g (0,0362 Mol, 72,5% der Theorie) 1-[N-(1-Chlormethyl-1-methyl-ethyl)-carbamoyl]-4-ethoxymethylenimino-3-methyl-1,2,4-triazolin-5-on vom Schmelzpunkt 77 °C.

6,1 g (0,02 Mol) obiger Verbindung werden in 50 ml absolutem Ethanol gelöst und bei 0 °C mit 2,3 g (0,06 Mol) Natriumborhydrid unter Rühren versetzt.

Nach 12 Stunden Rühren wird die Reaktionsmischung mit Salzsäure vorsichtig angesäuert, dann im Vakuum eingeengt und in Methylenchlorid aufgenommen. Nach Waschen mit Wasser, Trocknen und Einengen wird der Rückstand mit Ether/Petrolether verrieben.

Man erhält 1,9 g (0,0073 Mol, 36,3% der Theorie) 1-[N-(1-Chlormethyl-1-methyl-ethyl)-carbamoyl]-3-methyl-4-methylamino-1,2,4-triazolin-5-onvom Schmelzpunkt 93 °C.

Beispiel 3

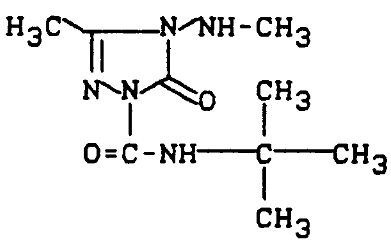

(Verfahren (c))

6,4 g (0,03 Mol) 4-Amino-1-[N-(1,1-dimethyl-ethyl)-carbamoyl]-3-methyl-1,2,4-triazolin-5-on werden in ca. 80 ml Orthoameisensäure-triethylester in Gegenwart von 0,1 g p-Toluolsulfonsäure für 2 Stunden unter Rückfluß erhitzt.

Die erkaltete Lösung wird im Vakuum eingeengt und der Rückstand in n-Hexan verrieben. Man erhält 4,9 g (0,0182 Mol, 60,7% der Theorie) 1-[N-(1,1-Dimethylethyl)-carbamoyl]-4-ethoxymethylenimino-3-methyl-1,2,4-triazolin-5-on vom Schmelzpunkt 102 °C.

2,7 g (0,01 Mol) obiger Verbindung werden in 40 ml absolutem Ethanol gelöst und bei 0 °C mit 0,8 g (0,011 Mol) Natriumborhydrid versetzt. Nach 1 Stunde bei 0 °C werden weitere 0,8 g Natriumborhydrid zugefügt.

Es wird 12 Stunden weitergerührt, im Vakuum eingeengt und in Methylenchlorid aufgenommen. Es wird mit verdünnter Salzsäure und Wasser neutral gewaschen, getrocknet und eingeengt.

Nach Verreiben des Rückstandes mit n-Hexan werden 1,0 g (0,0044 Mol, 44% der Theorie) 1-[N-(1,1-Dimethyl-ethyl)-carbamoyl]-3-methyl-4-methylamino-1,2,4-triazolin-5-on vom Schmelzpunkt 150 °C erhalten.

Beispiel 4

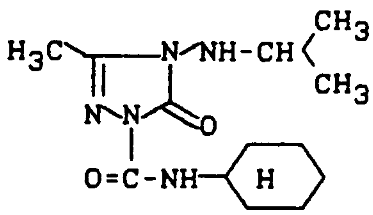

(Verfahren (f))

7,0 g (0,025 Mol) 1-(N-Cyclohexyl-carbamoyl)-4-isopropylidenimino-3-methyl-1,2,4-triazolin-5-on werden in 20 ml absolutem Methanol gelöst und unter Rühren mit 2 g (0,03 Mol) Natriumcyanborhydrid versetzt. Nach Zusatz einer Spatelspitze Methylorange wird methanolische Salzsäure zugetropft, bis ein Farbumschlag nach rot erfolgt (pH ca. 3). Die Lösung wird im Verlauf von 12 Stunden bei diesem pH-Wert gehalten, dann im Vakuum eingeengt und in Methylenchlorid aufgenommen. Nach Waschen mit Wasser, Trocknen und Einengen wird der Rückstand in Ether/ Petrolether verrieben.

Man erhält 3,1 g (0,011 Mol, 44% der Theorie) 1-(N-Cyclohexyl-carbamoyl)-4-isopropylamino-3-methyl-1,2,4-triazolin-5-on vom Schmelzpunkt 92 °C.

In entsprechender Weise und gemäß den allgemeinen Angaben zur Herstellung erhält man die folgenden substituierten Triazolinone der allgemeinen Formel (I):

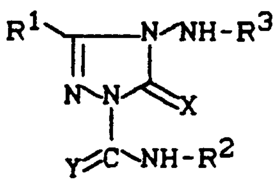

(I)

<u>Tabelle 1</u>

| Bsp. Nr. | $R^1$ | $R^2$ | $R^3$ | X | Y | Schmelzpunkt °C |
|---|---|---|---|---|---|---|
| 5 | $(CH_3)_2CH$ | $C(CH_3)_3$ | $CH_3$ | O | O | 121 |
| 6 | $(CH_3)_2CH$ | $C(CH_3)_2-CH_2Cl$ | $CH_3$ | O | O | 150 |
| 7 | $CH_3$ | $CH(CH_3)-CH_2Cl$ | $CH_3$ | O | O | 82 |
| 8 | $CH_3$ | 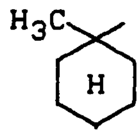 | $CH_3$ | O | O | 129 |
| 9 | $CH_3$ | 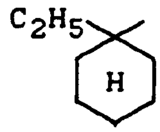 | $CH_3$ | O | O | 99 |
| 10 | $CH_3$ | $C(CH_3)_2-CH_2F$ | $CH_3$ | O | O | 161 |
| 11 | $(CH_3)_2CH$ | $C(CH_3)_2-CH_2F$ | $CH_3$ | O | O | 124 |
| 12 | $CH_3$ | 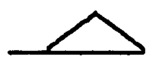 | $CH_3$ | O | O | 100 |
| 13 | $CH_3$ | $CH(CH_3)-C_3H_7-i$ | $CH_3$ | O | O | 132 |

| Bsp. Nr. | $R^1$ | $R^2$ | $R^3$ | X | Y | Schmelzpunkt °C |
|---|---|---|---|---|---|---|
| 14 | $CH_3$ | $C(CH_3)_2-C_4H_9$-n | $CH_3$ | O | O | 89 |
| 15 | $CH_3$ | 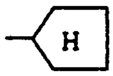 | $CH_3$ | O | O | 137 |
| 16 | $CH_3$ | 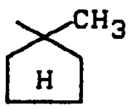 | $CH_3$ | O | O | 116 |
| 17 | $CH_3$ | n-$C_3H_7$ | $CH_3$ | O | O | 79 |
| 18 | $CH_3$ | i-$C_3H_7$ | $CH_3$ | O | O | 135 |
| 19 | $CH_3$ | $CH(CH_3)-C_2H_5$ | $CH_3$ | O | O | 127 |
| 20 | $CH_3$ | $C(CH_3)_2C{\equiv}CH$ | $CH_3$ | O | O | 98 |
| 21 | $CH_3$ | * $CH(CH_3)$- 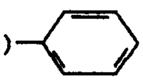 S-Konfiguration | $CH_3$ | O | O | 103 |
| 22 | $C_2H_5$ | $C(CH_3)_2CH_2Cl$ | $CH_3$ | O | O | 141 |
| 23 | $C_2H_5$ | $C(CH_3)_3$ | $CH_3$ | O | O | 105 |
| 24 | $C_2H_5$ | $C(CH_3)_2CH_2F$ | $CH_3$ | O | O | 107 |
| 25 | - | $C(CH_3)_3$ | $CH_3$ | O | O | 109 |
| 26 | H | $C(CH_3)_3$ | $CH_3$ | O | O | 112 |
| 27 | H | $C(CH_3)_2CH_2Cl$ | $CH_3$ | O | O | 95 |
| 28 | H | $C(CH_3)_2CH_2F$ | $CH_3$ | O | O | 116 |
| 29 | n-$C_3H_7$ | $C(CH_3)_3$ | $CH_3$ | O | O | 84 |
| 30 | n-$C_3H_7$ | $C(CH_3)_2CH_2Cl$ | $CH_3$ | O | O | 90 |
| 31 | n-$C_3H_7$ | $C(CH_3)_2CH_2F$ | $CH_3$ | O | O | 77 |
| 32 | $CH_3$ | $CH(CH_3)$- | $CH_3$ | O | O | 104 |
| 33 | $CH_3$ | $CH(CH_3)-CH_2OC_6H_5$ | $CH_3$ | O | O | 105 |

Herstellung der Ausgangsverbindungen

Beispiel IV-1

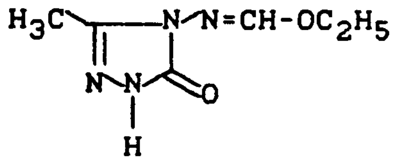

34,2 g (0,3 Mol) 4-Amino-3-methyl-1H-1,2,4-triazolin-5-on werden in 300 ml Orthoameisensäure-triethylester in Gegenwart von 0,1 g p-Toluolsulfonsäure für 2 Stunden unter Rückfluß erhitzt.

Die erkaltete Lösung wird im Vakuum eingeengt und der Rückstand mit Ether verrieben. Man erhält 43,3 g (0,255 Mol, 84,9% der Theorie) 4-Ethoxymethylenimino-3-methyl-1H-1,2,4-triazolin-5-on vom Schmelzpunkt 111 °C.

In entsprechender Weise und gemäß den allgemeinen Angaben zur Herstellung erhält man die folgenden Vorprodukte der allgemeinen Formel (IV):

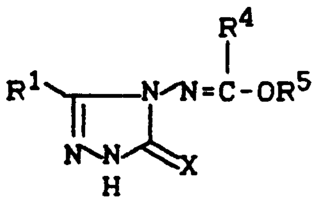

(IV)

Tabelle 2

| Bsp. Nr. | $R^1$ | $R^4$ | $R^5$ | X | Schmelzpunkt °C |
|---|---|---|---|---|---|
| IV-2 | $CH(CH_3)_2$ | H | $C_2H_5$ | O | 75 |
| IV-3 | $C_2H_5$ | H | $C_2H_5$ | O | |
| IV-4 | $CF_3$ | H | $C_2H_5$ | O | 77 |
| IV-5 | 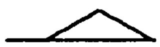 | H | $C_2H_5$ | O | 79 |
| IV-6 | $CH(CH_3)-C_2H_5$ | H | $C_2H_5$ | O | |
| IV-7 | $CH_2-O-CH_3$ | H | $C_2H_5$ | O | |
| IV-8 | $C_3H_7$-n | H | $C_2H_5$ | O | |
| IV-9 | $CH_3$ | $CH_3$ | $C_2H_5$ | O | 102 |
| IV-10 | $C_2H_5$ | $CH_3$ | $C_2H_5$ | O | 129 |
| IV-11 | $C_3H_7$-n | $CH_3$ | $C_2H_5$ | O | 75 |
| IV-12 | $CH_2-O-CH_3$ | $CH_3$ | $C_2H_5$ | O | |
| IV-13 | $CH(CH_3)-CH_3$ | $CH_3$ | $C_2H_5$ | O | 75 |
| IV-14 | 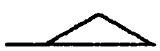 | $CH_3$ | $C_2H_5$ | O | 131 |
| IV-15 | $CF_3$ | $CH_3$ | $C_2H_5$ | O | 73 |
| IV-16 | $CH(CH_3)_2$ | $CH_3$ | $C_2H_5$ | O | 76 |

Tabelle 2

| Bsp. Nr. | $R^1$ | $R^4$ | $R^5$ | X | Schmelzpunkt °C |
|---|---|---|---|---|---|
| IV-17 | H | H | $C_2H_5$ | O | 124 |
| IV-18 | $CH_2OC_2H_5$ | H | $C_2H_5$ | O | 100 |
| IV-19 | H | $CH_3$ | $C_2H_5$ | O | 103 |
| IV-20 | $CH_2OC_2H_5$ | $CH_3$ | $C_2H_5$ | O | 83 |
| IV-21 | $CH_3$ | $C_2H_5$ | $C_2H_5$ | O | 96 |
| IV-22 | $C_2H_5$ | $C_2H_5$ | $C_2H_5$ | O | 92 |
| IV-23 | $CH(CH_3)_2$ | H | $C_2H_5$ | O | 86 |
| IV-24 | 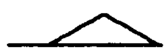 | $C_2H_5$ | $C_2H_5$ | O | 75 |
| IV-25 | $CH(CH_3)_2$ | $C_2H_5$ | $C_2H_5$ | O | 110 |
| IV-26 | $CH_3$ | H | $CH_3$ | O | 99 |

Analog Beispiel IV-1 können auch die Zwischenprodukte der allgemeinen Formel (V) hergestellt werden:

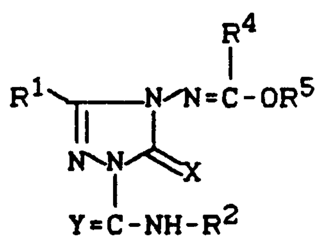

(V)

Tabelle 3

| Bsp. Nr. | $R^1$ | $R^2$ | $R^4$ | $R^5$ | X | Y | Schmelzpunkt °C |
|---|---|---|---|---|---|---|---|
| V-1 | $CH_3$ | $C(CH_3)_2-CH_2Cl$ | H | $C_2H_5$ | O | O | 77 |
| V-2 | $CH_3$ | $C(CH_3)_3$ | H | $C_2H_5$ | O | O | 102 |
| V-3 | $(CH_3)_2CH$ | $C(CH_3)_2-CH_2F$ | H | $C_2H_5$ | O | O | 88 |
| V-4 | $CH_3$ | $C(CH_3)_3$ | $CH_3$ | $C_2H_5$ | O | O | 108 |
| V-5 | $CH_3$ | $C(CH_3)_2-CH_2Cl$ | $CH_3$ | $C_2H_5$ | O | O | 134 |
| V-6 | $CH_3$ | $C(CH_3)_2-CH_2F$ | $CH_3$ | $C_2H_5$ | O | O | 105 |
| V-7 | $CH_3$ | $C_6H_5$ | H | $C_2H_5$ | O | O | 138 |
| V-8 | $CH(CH_3)_2$ | $C(CH_3)_3$ | H | $C_2H_5$ | O | O | 83 |
| V-9 | $CH(CH_3)_2$ | $C(CH_3)_2CH_2Cl$ | H | $C_2H_5$ | O | O | ($^1$H-NMR; $CDCl_3$, δ, ppm: 3,86 für $CH_2Cl$) |
| V-10 | $C_2H_5$ | $C(CH_3)_2CH_2Cl$ | H | $C_2H_5$ | O | O | ($^1$H-NMR, $CDl_3$, δ, ppm: 3,86 für $CH_2Cl$) |

Beispiel II-1

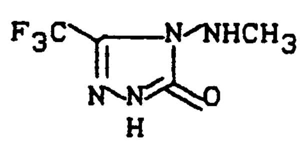

4,5 g (0,02 Mol) 4-Ethoxymethylenimino-3-trifluormethyl-1H-1,2,4-triazolin-5-on (Bsp. IV-4) werden in 20 ml Tetrahydrofuran gelöst und unter Rühren bei 20 °C zu einer Suspension von 1,0 g (0,026 Mol) Lithiumaluminiumhydrid in 80 ml Tetrahydrofuran getropft. Nach Ende der Gasentwicklung läßt man auf Raumtemperatur kommen und rührt noch 12 Stunden bei dieser Temperatur (20 °C).

Unter Eiskühlung wird dann zunächst eine Mischung aus 25 ml Wasser und 25 ml Tetrahydrofuran zugetropft und schließlich mit 250 ml Wasser verdünnt. Nach Ansäuren mit 2N-Salzsäure wird mit Essigester ausgeschüttelt, die organische Phase mit Natriumsulfat getrocknet und filtriert. Von Filtrat wird das Lösungsmittel im Wasserstrahlvakuum abdestilliert.

Man erhält 0,2 g (19 % der Theorie) 4-Methylamino-3-trifluormethyl-1H-1,2,4-triazolin-5-on als kristallinen Rückstand vom Schmelzpunkt 90 °C.

In entsprechender Weise erhält man die folgenden Ausgangsstoffe der allgemeinen Formel (II):

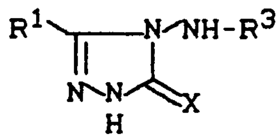

(II)

Tabelle 4

| Beispiel Nr. | $R^1$ | $R^3$ | X | Schmelzpunkt °C |
|---|---|---|---|---|
| II-2 | $CH_3$ | $CH_3$ | O | 113 |
| II-3 | $n-C_3H_7$ | $CH_3$ | O | 76 |
| II-4 | $CH(CH_3)_2$ | $CH_3$ | O | 105 |
| II-5 | 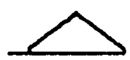 | $CH_3$ | O | 95 |
| II-6 | $C_2H_5$ | $CH_3$ | O | 101 |
| II-7 | H | $CH_3$ | O | 133 |

Anwendungsbeispiele:

In den folgenden Anwendungsbeispielen wurden die nachstehend aufgeführten Verbindungen als Vergleichsubstanzen eingesetzt:

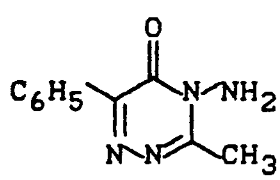

(A)

4-Amino-3-methyl-6-phenyl-1,2,4-triazin-5-on
(bekannt aus DE-OS 23 64 474, Beispiel I-22)

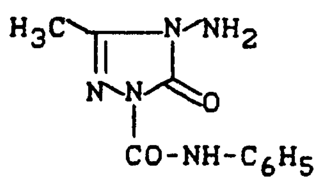

(B)

4-Amino-1-(N-phenylcarbamoyl)-3-methyl-triazolin-5-on
(bekannt aus EP-A 294 666, Beispiel 122)

Beispiel A

Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
Eine deutliche Überlegenheit in der Wirksamkeit bei der Bekämpfung von dikotylen Unkräutern gegenüber der Vergleichssubstanz (A) zeigen in diesem Test z.B. die Verbindungen gemäß den Herstellungsbeispielen: (3), (5) und (6).

Beispiel B

Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung
Eine deutliche Überlegenheit in der Wirksamkeit bei der Bekämpfung von dikotylen Unkräutern gegenüber den Vergleichssubstanzen (A) und (B) zeigen in diesem Test z.B. die Verbindungen gemäß den Herstellungsbeispielen: (3), (5) und (6).

Beispiel C

Erysiphe-Test (Gerste) / kurativ

| | |
|---|---|
| Lösungsmittel:<br>Emulgator: | 100 Gewichtsteile Dimethylformamid<br>0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine sehr gute Wirksamkeit zeigen bei diesem Test z.B. die Verbindungen gemäß folgender Herstellungsbeispiele: 9, 12, 15, 22 und 24.

Beispiel D

Erysiphe-Test (Weizen) / kurativ

| | |
|---|---|
| Lösungsmittel:<br>Emulgator: | 100 Gewichtsteile Dimethylformamid<br>0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine sehr gute Wirksamkeit zeigen bei diesem Test z.B. die Verbindungen gemäß folgender Herstellungsbeispiele: 9, 10, 12, 15, 22, 23 und 24.

**Patentansprüche**

**1.** Substituierte Triazolinone der allgemeinen Formel (I),

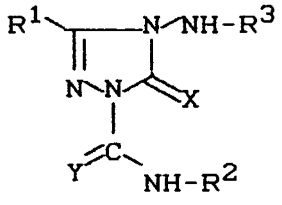

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkyl oder Cycloalkylalkyl mit jeweils 3 bis 7 Kohlenstoffatomen im Cycloalkylteil und gegebenenfalls 1 bis 6 Kohlenstoffatomen im geradkettigen oder verzweigten Alkylteil, für Tetrahydrofuranyl oder für Tetrahydrofuranylalkyl mit gegebenenfalls 1 bis 4 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, für Phenoxyalkyl mit 1 bis 4 Kohlenstoffatomen im Alkylteil, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl, bzw. Alkendiyl mit jeweils bis zu 4 Kohlenstoffatomen; $R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen -insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy, mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter -O-$CH_2$-O- Gruppe steht,

$R^3$ für $C_1$-$C_8$-Alkyl, $C_2$-$C_8$-Alkenyl, $C_3$-$C_6$-Cycloalkyl oder Phenyl-$C_1$-$C_4$-alkyl steht,

X für Sauerstoff oder Schwefel steht und

Y für Sauerstoff oder Schwefel steht,

**2.** Verfahren zur Herstellung von substituierten Triazolinonen der Formel (I)

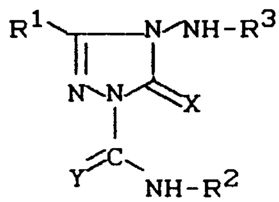

(I)

in welcher

$R^1$, $R^2$, $R^3$, $X^u$nd Y die gleichen Bedeutungen wie in Anspruch 1 haben,

dadurch gekennzeichnet, daß man

(a) 1H-Triazolinone der allgemeinen Formel (II)

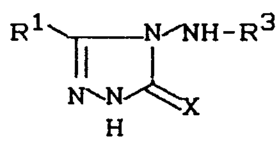

(II)

in welcher

$R^1$, $R^3$ und X die oben angegebene Bedeutung haben,

mit Iso(thio)cyanaten der allgemeinen Formel (III)

$R^2$-N = C = Y (III)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder daß man

(b) 1H-Triazolinone der allgemeinen Formel (IV)

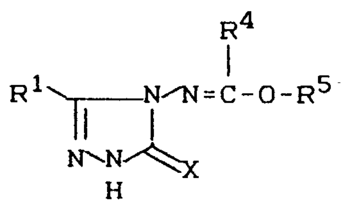

(IV)

in welcher

$R^1$ und X die oben angegebene Bedeutung haben und

$R^4$ für Wasserstoff, $C_1$-$C_4$-Alkyl, $C_2$-$C_4$-Alkenyl, $C_2$-$C_4$-Alkinyl, $C_3$-$C_6$-Cycloalkyl, Phenyl oder Benzyl steht,

$R^5$ für $C_1$-$C_4$-Alkyl, $C_2$-$C_4$-Alkenyl, $C_2$-$C_4$-Alkinyl, $C_3$-$C_6$-Cycloalkyl, Phenyl oder Benzyl steht,

mit Iso(thio)cyanaten der allgemeinen Formel (III)

$R^2$-N = C = Y (III)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt
und die so erhaltenen Verbindungen der allgemeinen Formel (V)

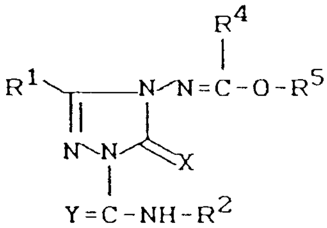

(V)

in welcher
$R^1$, $R^2$, $R^4$, $R^5$, X und Y die oben angegebene Bedeutung haben,
in einem zweiten Reaktionsschritt mit einem Reduktionsmittel der Formel (VI) oder (VII)

$MBH_4$ (VI)

$MAlH_4$ (VII)

in welchem
M für ein Alkalimetallatom steht,
gegebenenfalls in Gegenwart eines Verdünnungsmittels zu den Verbindungen der allgemeinen Formel (VIII)

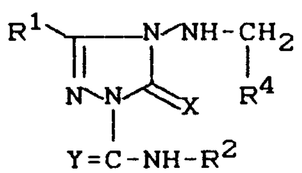

(VIII)

in welcher
$R^1$, $R^2$, $R^4$, X und Y die oben angegebene Bedeutung haben,
umsetzt,
oder daß man
(c) 1-Carbamoyltriazolinone der allgemeinen Formel (IX)

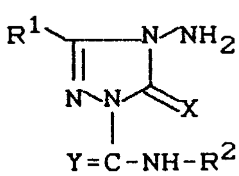

(IX)

in welcher
$R^1$, $R^2$, X und Y die oben angegebene Bedeutung haben
mit Orthocarbonsäureestern der allgemeinen Formel (X)

$R^4\text{-}C(OR^5)_3$ (X)

in welcher

$R^4$ und $R^5$ die oben angegebene Bedeutung haben,
gegebenenfalls in Gegenwart eines Verdünnungsmittels und gegebenenfalls in Gegenwart eines Reaktionshilfsmittels zu Verbindungen der allgemeinen Formel (V)

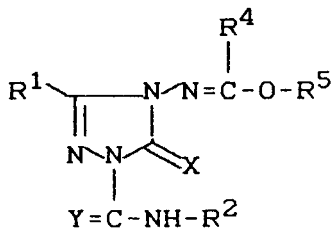

(V)

in welcher
$R^1$, $R^2$, $R^4$, $R^5$, X und Y die oben angegebene Bedeutung haben,
umsetzt und diese Verbindungen der Formel (V) in einem zweiten Reaktionsschritt mit einem Reduktionsmittel der Formel (VI) oder (VII)

$MBH_4$ (VI)

$MAlH_4$ (VII)

in welchem
M für ein Alkalimetallatom steht,
gegebenenfalls in Gegenwart eines Verdünnungsmittels zu den Verbindungen der allgemeinen Formel (VIII)

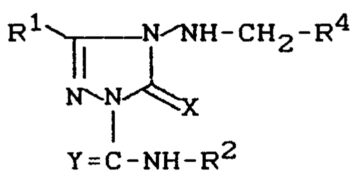

(VIII)

in welcher
$R^1$, $R^2$, $R^4$, X und Y die oben angegebene Bedeutung haben,
umsetzt,
oder daß man
(d) 1H-Triazolinone der allgemeinen Formel (II)

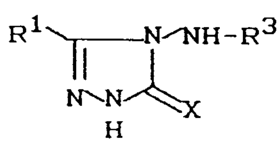

(II)

in welcher
$R^1$, $R^3$ und X die oben angegebene Bedeutung haben,
mit (Thio)Chlorameisensäureestern der allgemeinen Formel (XI)

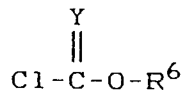

(XI)

in welcher

$R^6$ für $C_1$-$C_4$-Alkyl, Phenyl oder Benzyl steht und

Y die oben angegebene Bedeutung hat,

gegebenenfalls in Gegenwart eines Verdünnungsmittels und gegebenenfalls in Gegenwart eines Reaktionshilfsmittels umsetzt und die so erhältlichen Triazolinone der allgemeinen Formel (XII)

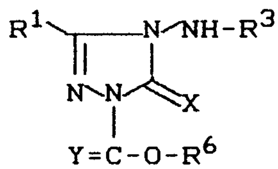

(XII)

in welcher

$R^1$, $R^3$, $R^6$, X und Y die oben angegebene Bedeutung haben,

in einer anschließenden 2. Stufe mit Aminen der allgemeinen Formel (XIII)

$R^2$-$NH_2$ (XIII)

in welcher

$R^2$ die oben angegebene Bedeutung hat,

gegebenenfalls in Gegenwart eines Verdünnungsmittels und gegebenenfalls in Gegenwart eines Reaktionshilfsmittels zu Verbindungen der Formel (I) umsetzt,

oder daß man

(e) 1H-Triazolinone der allgemeinen Formel (II)

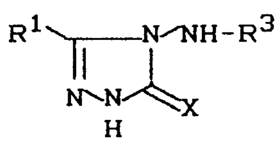

(II)

in welcher

$R^1$, $R^3$ und X die oben angegebene Bedeutung haben,

mit (Thio)Urethanen der allgemeinen Formel (XIV)

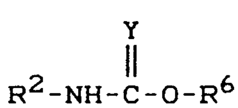

(XIV)

in welcher

$R^2$, $R^6$ und Y die oben angegebene Bedeutung haben,

gegebenenfalls in Gegenwart eines Verdünnungsmittels und gegebenenfalls in Gegenwart eines Reaktionshilfsmittels umsetzt,

oder daß man

(f) Triazolinon-Hydrazone der allgemeinen Formel (XV)

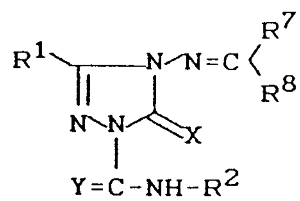

(XV)

in welcher

| | |
|---|---|
| $R^1$, $R^2$, X und Y | die oben angegebene Bedeutung haben und, |
| $R^7$ und $R^8$ | jeweils unabhängig voneinander für Wasserstoff, $C_1$-$C_4$-Alkyl, Phenyl oder Benzyl stehen, |

mit Reduktionsmitteln gegebenenfalls in Gegenwart von Katalysatoren und gegebenenfalls in Gegenwart von Verdünnungsmitteln umsetzt.

**3.** Herbizide und fungizide Mittel, gekennzeichnet durch einen Gehalt an mindestens einem substituierten Triazolinon der Formel (I) gemäß Anspruch 1 oder 2.

**4.** Verfahren zur Bekämpfung von unerwünschten Pflanzen und Pilzen, dadurch gekennzeichnet, daß man substituierte Triazolinone der Formel (I) gemäß Anspruch 1 oder 2 auf die unerwünschten Pflanzen bzw. Pilze und/oder ihren Lebensraum einwirken läßt.

**5.** Verwendung von substituierten Triazolinonen der Formel (I) gemäß Anspruch 1 oder 2 zur Bekämpfung von unerwünschten Pflanzen und Pilzen.

**6.** Verfahren zur Herstellung von herbiziden und fungiziden Mitteln, dadurch gekennzeichnet, daß man substituierte Triazolinone der Formel (I) gemäß Anspruch 1 oder 2 mit Streckmitteln und/oder oberflächenaktiven Substanzen vermischt.

**7.** Verbindungen der Formel (V)

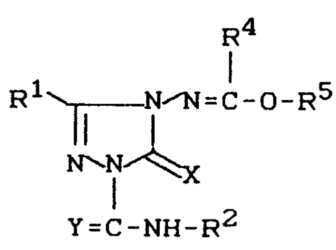

(V)

in welcher

| | |
|---|---|
| $R^1$, $R^2$, X und Y | die in Anspruch 1 angegebenen Bedeutungen haben und |
| $R^4$ | für Wasserstoff, $C_1$-$C_4$-Alkyl, $C_2$-$C_4$-Alkenyl, $C_2$-$C_4$-Alkinyl, $C_3$-$C_6$-Cycloalkyl, Phenyl oder Benzyl steht und |
| $R^5$ | für $C_1$-$C_4$-Alkyl, $C_2$-$C_4$-Alkenyl, $C_2$-$C_4$-Alkinyl, $C_3$-$C_6$-Cycloalkyl, Phenyl oder Benzyl steht. |

**8.** Triazolinone der Formel (XII)

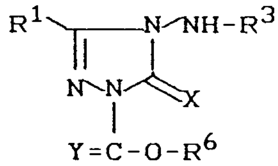

(XII)

in welcher

| | |
|---|---|
| $R^1$, $R^3$, X und Y | die in Anspruch 1 angegebenen Bedeutungen haben, und |
| $R^6$ | für $C_1$-$C_4$-Alkyl, Phenyl oder Benzyl steht. |

**9.** 1H-Triazolinone der Formel (II)

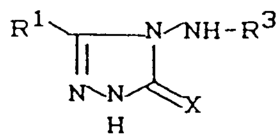

(II)

in welcher

| | |
|---|---|
| $R^1$ | für Ethyl, n- oder i-Propyl, n-, i-, s-, oder t-Butyl, n- oder i-Pentyl, n- oder i-Hexyl, Cyclopropyl, Cyclopentyl oder Cyclohexyl steht, |
| $R^3$ | für Methyl steht und |
| X | für Sauerstoff steht. |

**10.** 1H-Triazolinone der Formel (II)

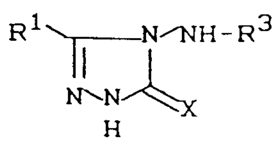

(II)

in welcher

| | |
|---|---|
| $R^1$ | für Methyl, Ethyl, n- oder i-Propyl, n-, i-, s- oder t-Butyl, n- oder i-Pentyl, n- oder i-Hexyl, Cyclopropyl, Cyclopentyl oder Cyclohexyl steht, |
| $R^3$ | für Ethyl, n- oder i-Propyl, n-, oder t-Butyl steht, |
| X | für Sauerstoff steht. |

## Claims

1. Substituted triazolinones of the general formula (I)

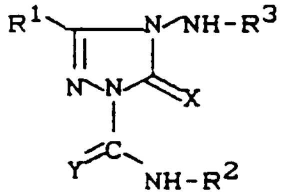

(I)

in which

$R^1$ represents hydrogen, or represents in each case a straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms or alkoxyalkyl having 1 to 6 carbon atoms in the individual alkyl moieties, or represents cycloalkyl or cycloalkylalkyl, each of which has 3 to 7 carbon atoms in the cycloalkyl moiety and if appropriate 1 to 6 carbon atoms in the straight-chain or branched alkyl moiety, or represents tetrahydrofuranyl, or represents tetrahydrofuranylalkyl, if appropriate having 1 to 4 carbon atoms in the straight-chain or branched alkyl moiety, or represents aralkyl or aryl, each of which has 6 to 10 carbon atoms in the aryl moiety and where appropriate 1 to 6 carbon atoms in the straight-chain or branched alkyl moiety and each of which is optionally monosubstituted or polysubstituted by identical or different substituents, suitable aryl substituents in each case being: halogen, cyano, nitro and in each case straight-chain or branched alkyl, alkoxy, alkylthio, halogenoalkyl, halogenoalkoxy or halogenoalkylthio, each of which has 1 to 4 carbon atoms and where appropriate 1 to 9 identical or different halogen atoms,

$R^2$ represents hydrogen, or represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15, or 13, identical or different halogen atoms, cyanoalkyl having 1 to 8 carbon atoms or hydroxyalkyl having 1 to 8 carbon atoms and 1 to 6 hydroxyl groups, or represents phenoxyalkyl having 1 to 4 carbon atoms in the alkyl moiety, or alkoxyalkyl, alkoxycarbonylalkyl or alkoxycarbonylalkenyl, each of which has up to 6 carbon atoms in the individual alkyl or alkenyl moieties, or alkylaminoalkyl or dialkylaminoalkyl, each of which has 1 to 6 carbon atoms in the individual alkyl moieties, or represents cycloalkyl, cycloalkylalkyl, cycloalkenyl or cycloalkenylalkyl, each of which has 3 to 8 carbon atoms in the cycloalkyl or cycloalkenyl moiety and where appropriate 1 to 6 carbon atoms in the alkyl moiety and each of which is optionally monosubstituted or polysubstituted by identical or different substituents, suitable substituents in each case being: halogen, cyano and in each case straight-chain or branched alkyl or halogenoalkyl, each of which has 1 to 4 carbon atoms and if appropriate 1 to 9 identical or different halogen atoms, or straight-chain or branched halogenoalkyl having up to 4 carbon atoms and 1 to 5 identical or different halogen atoms, or alkanediyl, or alkenediyl, each of which has up to 4 carbon atoms and each of which is double-linked; $R^2$ furthermore represents heterocyclylalkyl which has 1 to 6 carbon atoms in the straight-chain or branched alkyl moiety and 1 to 9 carbon atoms and 1 to 3 hetero atoms - in particular nitrogen, oxygen and/or sulphur - in the heterocyclyl moiety and which is optionally monosubstituted or polysubstituted by identical or different substituents in the heterocyclyl moiety, suitable substituents in each case being: halogen, cyano, nitro and in each case straight-chain or branched alkyl, alkoxy, alkylthio, halogenoalkyl, halogenoalkoxy, halogenoalkylthio or alkoxycarbonyl, each of which has 1 to 5 carbon atoms and where appropriate 1 to 9 identical or different halogen atoms; $R^2$ furthermore represents in each case straight-chain or branched alkoxy having 1 to 8 carbon atoms, alkenyloxy having 2 to 8 carbon atoms or alkinyloxy

having 2 to 8 carbon atoms, and finally represents aralkyl, aroyl, aryl, aralkyloxy or aryloxy, each of which has 6 to 10 carbon atoms in the aryl moiety and where appropriate 1 to 8 carbon atoms in the straight-chain or branched alkyl moiety and each of which is optionally monosubstituted or polysubstituted by identical or different substituents, suitable alkyl substituents, where appropriate, being halogen and cyano and suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms in the alkyl moiety and where appropriate 1 to 9 identical or different halogen atoms, or cycloalkyl having 3 to 6 carbon atoms, and phenoxy; or $R^2$ represents benzyl having an $-O-CH_2-O-$ group which is fused in the phenyl moiety,

$R^3$ represents $C_1$-$C_8$-alkyl, $C_2$-$C_8$-alkenyl, $C_3$-$C_6$-cycloalkyl or phenyl-$C_1$-$C_4$-alkyl,

X represents oxygen or sulphur and

Y represents oxygen or sulphur.

**2.** Process for the preparation of substituted triazolinones of the formula (I)

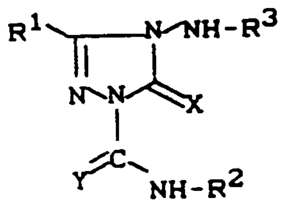

(I)

in which

$R^1$, $R^2$, $R^3$, X and Y have the same meanings as in Claim 1,

characterized in that

(a) 1H-triazolinones of the general formula (II)

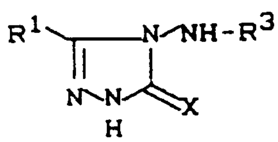

(II)

in which

$R^1$, $R^3$ and X have the abovementioned meaning,

are reacted with iso(thio)cyanates of the general formula (III)

$R^2$-N = C = Y (III)

in which

$R^2$ and Y have the abovementioned meaning,

if appropriate in the presence of a diluent and if appropriate in the presence of a reaction auxiliary, or in that

(b) 1H-triazolinones of the general formula (IV)

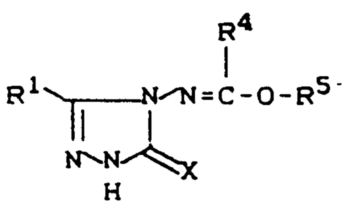

(IV)

in which

- $R^1$ and X have the abovementioned meaning and
- $R^4$ represents hydrogen, $C_1$-$C_4$-alkyl, $C_2$-$C_4$-alkenyl, $C_2$-$C_4$-alkinyl, $C_3$-$C_6$-cycloalkyl, phenyl or benzyl,
- $R^5$ represents $C_1$-$C_4$-alkyl, $C_2$-$C_4$-alkenyl, $C_2$-$C_4$-alkinyl, $C_3$-$C_6$-cycloalkyl, phenyl or benzyl,

are reacted with iso(thio)cyanates of the general formula (III)

$R^2$-N=C=Y (III)

in which

- $R^2$ and Y have the abovementioned meaning,

if appropriate in the presence of a diluent and if appropriate in the presence of a reaction auxiliary and the resulting compounds of the general formula (V)

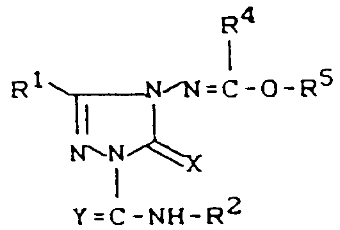

(V)

in which

- $R^1$, $R^2$, $R^4$, $R^5$, X and Y have the abovementioned meaning,

are reacted in a second reaction step with a reducing agent of the formula (VI) or (VII)

$MBH_4$ (VI)

$MA1H_4$ (VII)

in which

- M represents an alkali metal atom,

if appropriate in the presence of a diluent, to give the compounds of the general formula (VIII)

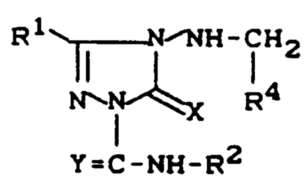

(VIII)

in which

- $R^1$, $R^2$, $R^4$, X and Y have the abovementioned meaning,

or in that

(c) 1-carbamoyltriazolinones of the general formula (IX)

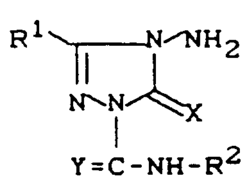

(IX)

in which

$R^1$, $R^2$, X and Y have the abovementioned meaning,

are reacted with orthocarboxylic acid esters of the general formula (X)

$R^4-C(OR^5)_3$ (X)

in which

$R^4$ and $R^5$ have the abovementioned meaning,

if appropriate in the presence of a diluent and if appropriate in the presence of a reaction auxiliary, to give compounds of the general formula (V)

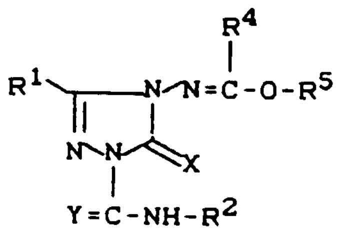

(V)

in which

$R^1$, $R^2$, $R^4$, $R^5$, X and Y have the abovementioned meaning,

and these compounds of the formula (V) are reacted in a second reaction step with a reducing agent of the formula (VI) or (VII)

$MBH_4$ (VI)

$MA1H_4$ (VII)

in which

M represents an alkali metal atom,

if appropriate in the presence of a diluent, to give the compounds of the general formula (VIII)

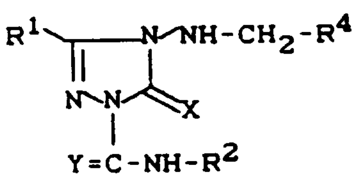

(VIII)

in which

$R^1$, $R^2$, $R^4$, X and Y have the abovementioned meaning,

or in that

(d) 1H-triazolinones of the general formula (II)

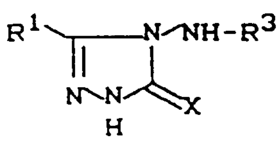

(II)

in which

$R^1$, $R^3$ and X have the abovementioned meaning,

are reacted with (thio)chloroformic acid esters of the general formula (XI)

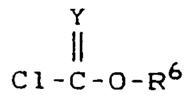

(XI)

in which

$R^6$ represents $C_1$-$C_4$-alkyl, phenyl or benzyl and

Y has the abovementioned meaning,

if appropriate in the presence of a diluent and if appropriate in the presence of a reaction auxiliary, and the resulting triazolinones of the general formula (XII)

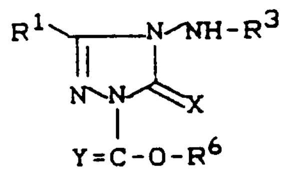

(XII)

in which

$R^1$, $R^3$, $R^6$, X and Y have the abovementioned meaning,

are reacted in a subsequent 2nd step with amines of the general formula (XIII)

$R^2$-$NH_2$ (XIII)

in which

$R^2$ has the abovementioned meaning,

if appropriate in the presence of a diluent and if appropriate in the presence of a reaction auxiliary to give compounds of the formula (I), or in that

(e) 1H-triazolinones of the general formula (II)

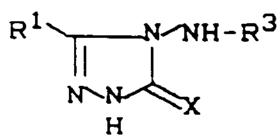

(II)

in which

$R^1$, $R^3$ and X have the abovementioned meaning,

are reacted with (thio)urethanes of the general formula (XIV)

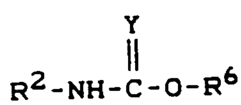

(XIV)

in which

$R^2$, $R^6$ and Y have the abovementioned meaning,

if appropriate in the presence of a diluent and if appropriate in the presence of a reaction auxiliary, or in that

(f) triazolinone-hydrazones of the general formula (XV)

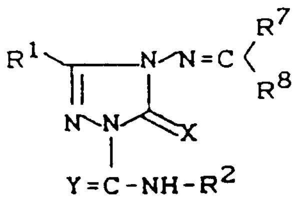

(XV)

in which

$R^1$, $R^2$, X and Y have the abovementioned meaning and

$R^7$ and $R^8$ in each case independently of one another represent hydrogen, $C_1$-$C_4$-alkyl, phenyl or benzyl,

are reacted with reducing agents, if appropriate in the presence of catalysts and if appropriate in the presence of diluents.

**3.** Herbicidal and fungicidal agents, characterized in that they contain at least one substituted triazolinone of the formula (I) according to Claim 1 or 2.

**4.** Method for controlling undesired plants and fungi, characterized in that substituted triazolinones of the formula (I) according to Claim 1 or 2 are allowed to act on the undesired plants or fungi and/or their environment.

**5.** Use of substituted triazolinones of the formula (I) according to Claim 1 or 2 for controlling undesired plants and fungi.

**6.** Process for the preparation of herbicidal and fungicidal agents, characterized in that substituted triazolinones of the formula (I) according to Claim 1 or 2 are mixed with extenders and/or surface-active substances.

**7.** Compounds of the formula (V)

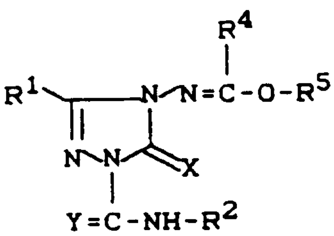

(V)

in which

$R^1$, $R^2$, X and Y have the meanings indicated in Claim 1 and

$R^4$ represents hydrogen, $C_1$-$C_4$-alkyl, $C_2$-$C_4$-alkenyl, $C_2$-$C_4$-alkinyl, $C_3$-$C_6$-cycloalkyl, phenyl or benzyl and

$R^5$ represents $C_1$-$C_4$-alkyl, $C_2$-$C_4$-alkenyl, $C_2$-$C_4$-alkinyl, $C_3$-$C_6$-cycloalkyl, phenyl or benzyl.

**8.** Triazolinones of the formula (XII)

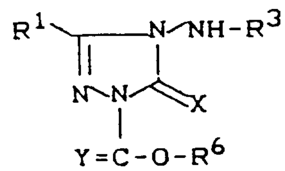

(XII)

in which

$R^1$, $R^3$, X and Y have the meanings indicated in Claim 1 and
$R^6$ represents $C_1$-$C_4$-alkyl, phenyl or benzyl.

**9.** 1H-Triazolinones of the formula (II)

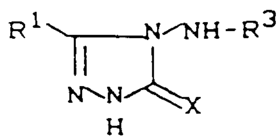

(II)

in which

$R^1$ represents ethyl, n- or i-propyl, n-, i-, s- or t-butyl, n- or i-pentyl, n- or i-hexyl, cyclopropyl, cyclopentyl or cyclohexyl,
$R^3$ represents methyl and
X represents oxygen.

**10.** 1H-Triazolinones of the formula (II)

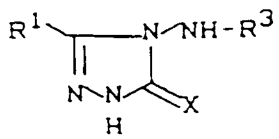

(II)

in which

$R^1$ represents methyl, ethyl, n- or i-propyl, n-, i-, s- or t-butyl, n- or i-pentyl, n- or i-hexyl, cyclopropyl, cyclopentyl or cyclohexyl,
$R^3$ represents ethyl, n- or i-propyl, n- or i-butyl, and
X represents oxygen.

## Revendications

**1.** Triazolinones substituées répondant à la formule générale (I)

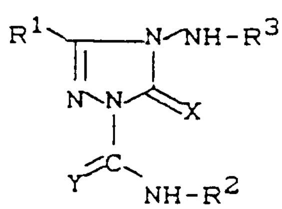

( I )

dans laquelle

$R^1$ représente un atome d'hydrogène, un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 2 à 8 atomes de carbone et de 1 à 13 atomes d'halogène identiques ou différents, un groupe alcoxyalkyle contenant de 1 à 6 atomes de carbone dans les fractions alkyle individuelles, chacun de ces groupes étant à chaîne droite ou ramifiée; un groupe cycloalkyle ou un groupe cycloalkylalkyle contenant chacun de 3 à 7 atomes de carbone dans la fraction cycloalkyle et éventuellement de 1 à 6 atomes de carbone dans la fraction alkyle à chaîne droite ou ramifiée, un groupe tétrahydrofuranyle ou un groupe tétrahydrofuranylalkyle contenant éventuellement de 1 à 4 atomes de carbone dans la fraction alkyle à chaîne droite ou ramifiée; ou encore un groupe aralkyle ou un groupe aryle contenant chacun de 6 à 10 atomes de carbone dans la fraction aryle et éventuellement de 1 à 6 atomes de carbone dans la fraction alkyle à chaîne droite ou ramifiée, chacun de ces groupes étant éventuellement substitué une ou plusieurs fois de manière identique ou différente, dans lesquels, comme substituants du groupe aryle, entrent chaque fois en ligne de compte : un atome d'halogène, un groupe cyano, un groupe nitro, ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée,

$R^2$ représente un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, chacun de ces groupes étant à chaîne droite ou ramifiée, un groupe phénoxyalkyle contenant de 1 à 4 atomes de carbone dans la fraction alkyle, un groupe alcoxyalkyle, un groupe alcoxycarbonylalkyle ou un groupe alcoxycarbonylalcényle contenant chacun jusqu'à 6 atomes de carbone dans les fractions individuelles alkyle ou alcényle, un groupe alkylaminoalkyle ou dialkylaminoalkyle contenant chacun de 1 à 6 atomes de carbone dans les fractions alkyle individuelles, un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou cycloalcényle et éventuellement de 1 à 6 atomes de carbone dans la fraction alkyle, ces groupes étant éventuellement substitués une ou plusieurs fois de manière identique ou différente, dans lesquels, comme substituants, entrent chaque fois en ligne de compte : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou bien un groupe halogénalcényle à chaîne droite ou ramifiée contenant jusqu'à 4 atomes de carbone et de 1 à 5 atomes d'halogène identiques ou différents ou encore un groupe alcanediyle ou alcènediyle contenant chacun jusqu'à 4 atomes de carbone et étant chacun à liaison double; $R^2$ représente, en outre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéro-atomes - en particulier un atome d'azote, un atome d'oxygène et/ou un atome de soufre - dans la fraction hétérocyclyle, éventuellement substitué une ou plusieurs fois de manière identique ou différente dans la fraction hétérocyclyle, dans lesquels, comme substituants, entrent en ligne de compte : un atome d'halogène, un groupe cyano, un groupe nitro, ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; $R^2$ représente, en outre, un groupe alcoxy contenant de 1 à 8 atomes de carbone, un groupe alcényloxy contenant de 2 à 8

atomes de carbone ou un groupe alcynyloxy contenant de 2 à 8 atomes de carbone, chacun étant à chaîne droite ou ramifiée; et enfin un groupe aralkyle, un groupe aroyle, un groupe aryle, un groupe aralkyloxy ou un groupe aryloxy contenant chacun de 6 à 10 atomes de carbone dans la fraction aryle et éventuellement de 1 à 8 atomes de carbone dans la fraction alkyle à chaîne droite ou ramifiée, chacun de ces groupes étant éventuellement substitué une ou plusieurs fois de manière identique ou différente, dans lesquels, comme substituants du groupe alkyle, on envisage éventuellement un atome d'halogène et un groupe cyano, et dans lesquels, comme substituants du groupe aryle, on envisage chaque fois : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone dans la fraction alkyle et éventuellement de 1 à 9 atomes d'halogène identiques ou différents, chacun de ces groupes étant à chaîne droite ou ramifiée; un groupe cycloalkyle contenant de 3 à 6 atomes de carbone et un groupe phénoxy; ou encore $R^2$ représente un groupe benzyle contenant un groupe $-O-CH_2-O-$ condensé dans la fraction phényle,

$R^3$ représente un groupe alkyle en $C_1$-$C_8$, un groupe alcényle en $C_2$-$C_8$, un groupe cycloalkyle en $C_3$-$C_6$ ou un groupe phénylalkyle en $C_1$-$C_4$,

X représente un atome d'oxygène ou un atome de soufre et

Y représente un atome d'oxygène ou un atome de soufre.

**2.** Procédé pour la préparation de triazolinones substituées répondant à la formule (I)

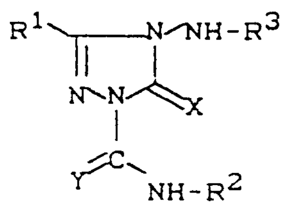

(I)

dans laquelle

$R^1$, $R^2$, $R^3$, X et Y ont les mêmes significations que celles indiquées à la revendication 1,

caractérisé en ce qu'on fait réagir

(a) des 1H-triazolinones répondant à la formule générale (II)

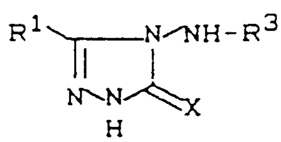

(II)

dans laquelle

$R^1$, $R^3$ et X ont la signification indiquée ci-dessus,

avec des iso(thio)cyanates répondant à la formule générale (III)

$R^2-N=C=Y$ (III)

dans laquelle

$R^2$ et Y ont la signification indiquée ci-dessus,

éventuellement en présence d'un diluant et éventuellement en présence d'un adjuvant réactionnel,

ou bien en ce qu'on fait réagir

(b) des 1H-triazolinones répondant à la formule générale (IV)

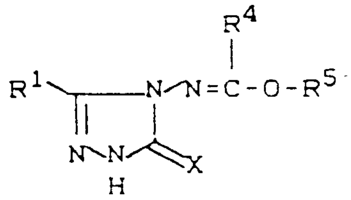

(IV)

dans laquelle

$R^1$ et X ont la signification indiquée ci-dessus, et

$R^4$ représente un atome d'hydrogène, un groupe alkyle en $C_1$-$C_4$, un groupe alcényle en $C_2$-$C_4$, un groupe alcynyle en $C_2$-$C_4$, un groupe cycloalkyle en $C_3$-$C_6$, un groupe phényle ou un groupe benzyle,

$R^5$ représente un groupe alkyle en $C_1$-$C_4$, un groupe alcényle en $C_2$-$C_4$, un groupe alcynyle en $C_2$-$C_4$, un groupe cycloalkyle en $C_3$-$C_6$, un groupe phényle ou un groupe benzyle,

avec des iso(thio)cyanates répondant à la formule générale (III)

$R^2$-N = C = Y (III)

dans laquelle

$R^2$ et Y ont la signification indiquée ci-dessus,

éventuellement en présence d'un diluant et éventuellement en présence d'un adjuvant réactionnel, et on fait réagir les composés ainsi obtenus répondant à la formule générale (V)

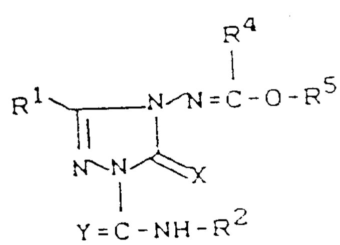

(V)

dans laquelle

$R^1$, $R^2$, $R^4$, $R^5$, X et Y ont la signification indiquée ci-dessus,

dans une deuxième étape réactionnelle avec un agent de réduction de formule (VI) ou (VII)

$MBH_4$ (VI)

$MAlH_4$ (VII)

dans laquelle

M représente un atome de métal alcalin,

éventuellement en présence d'un diluant pour obtenir les composés répondant à la formule générale (VIII)

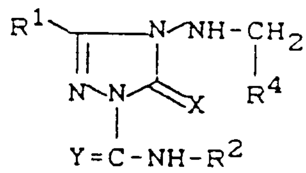

(VIII)

dans laquelle

$R^1$, $R^2$, $R^4$, X et Y ont la slgnification indiquée ci-dessus,

ou bien en ce qu'on fait réagir

(c) des 1-carbamoyltriazolinones répondant à la formule générale (IX)

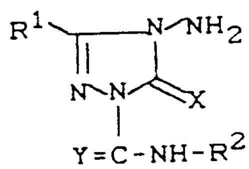

(IX)

dans laquelle

$R^1$, $R^2$, X et Y ont la signification indiquée ci-dessus,

avec des esters orthocarboxyliques répondant à la formule générale (X)

$R^4$-C($OR^5$)$_3$ (X)

dans laquelle

$R^4$ et $R^5$ ont la signification indiquée ci-dessus,

éventuellement en présence d'un diluant et éventuellement en présence d'un adjuvant réactionnel pour obtenir les composés répondant à la formule générale (V)

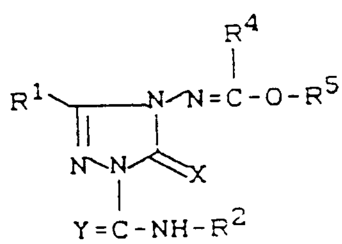

(V)

dans laquelle

$R^1$, $R^2$, $R^4$, $R^5$, X et Y ont la même signification que celle indiquée ci-dessus,

et on fait réagir ces composés de formule (V) dans une deuxième étape réactionnelle avec un agent de réduction de formule (VI) ou (VII)

$MBH_4$ (VI)

$MAlH_4$ (VII)

dans laquelle

M représente un atome de métal alcalin,

éventuellement en présence d'un diluant pour obtenir les composés répondant à la formule générale (VIII)

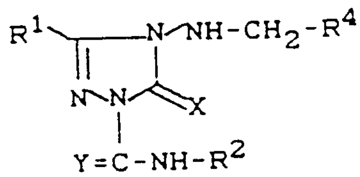

(VIII)

dans laquelle

$R^1$, $R^2$, $R^4$, X et Y ont la signification indiquée ci-dessus,

ou bien en ce qu'on fait réagir

(d) des 1H-triazolinones répondant à la formule générale (II)

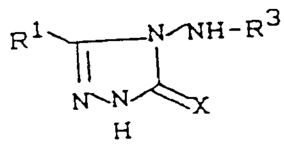

(II)

dans laquelle

$R^1$, $R^3$ et X ont la signification indiquée ci-dessus,

avec des esters (thio)chloroformiques répondant à la formule générale (XI)

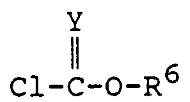

(XI)

dans laquelle

$R^6$ représente un groupe alkyle en $C_1$-$C_4$, un groupe phényle ou un groupe benzyle, et

Y a la signification indiquée ci-dessus,

éventuellement en présence d'un diluant et éventuellement en présence d'un adjuvant réactionnel, et on fait réagir les triazolinones ainsi obtenues répondant à la formule générale (XII)

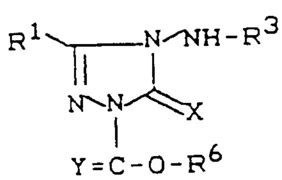

(XII)

dans laquelle

$R^1$, $R^3$, $R^6$, X et Y ont la signification indiquée ci-dessus,

dans une deuxième étape ultérieure avec des amines répondant à la formule générale (XIII)

$R^2$-$NH_2$ (XIII)

dans laquelle

$R^2$ a la signification indiquée ci-dessus,

éventuellement en présence d'un diluant et éventuellement en présence d'un adjuvant réactionnel pour obtenir des composés de formule (I),

ou bien en ce qu'on fait réagir

(e) des 1H-triazolinones répondant à la formule générale (II)

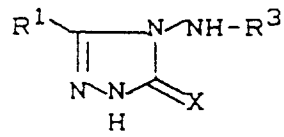

(II)

dans laquelle

$R^1$, $R^3$ et X ont la signification indiquée ci-dessus,

avec des (thio)uréthannes répondant à la formule générale (XIV)

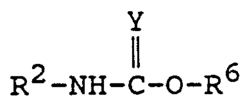

(XIV)

dans laquelle

$R^2$, $R^6$ et Y ont la signification indiquée ci-dessus,

éventuellement en présence d'un diluant et éventuellement en présence d'un adjuvant réactionnel, ou bien en ce qu'on fait réagir

(f) des triazolinone-hydrazones répondant à la formule générale (XV)

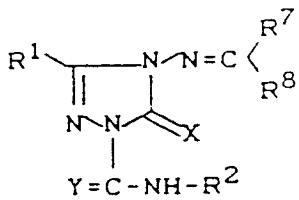

(XV)

dans laquelle

$R^1$, $R^2$, X et Y ont la signification indiquée ci-dessus, et

$R^7$ et $R^8$ représentent chacun, indépendamment l'un de l'autre, un atome d'hydrogène, un groupe alkyle en $C_1$-$C_4$, un groupe phényle ou un groupe benzyle,

avec des agents de réduction, éventuellement en présence de catalyseurs et éventuellement en présence de diluants.

**3.** Agents herbicides et fongicides, caractérisés par une teneur en au moins une triazolinone substituée de formule (I) selon la revendication 1 ou 2.

**4.** Procédé pour lutter contre des plantes et des champignons non désirés, caractérisé en ce qu'on laisse agir des triazolinones substituées de formule (I) selon la revendication 1 ou 2 sur les plantes ou les champignons non désirés et/ou sur leur biotope.

**5.** Utilisation de triazolinones substituées de formule (I) selon la revendication 1 ou 2, pour lutter contre des plantes et des champignons non désirés.

**6.** Procédé pour la préparation d'agents herbicides et fongicides, caractérisé en ce qu'on mélange des triazolinones substituées de formule (I) selon la revendication 1 ou 2, avec des diluants et/ou des substances tensioactives.

**7.** Composés de formule (V)

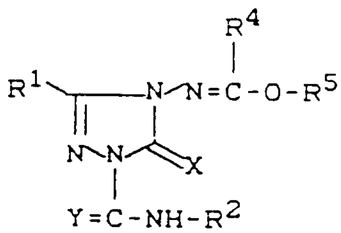

(V)

dans laquelle

$R^1$, $R^2$, X et Y ont les significations indiquées à la revendication 1 et

$R^4$ représente un atome d'hydrogène, un groupe alkyle en $C_1$-$C_4$, un groupe alcényle en $C_2$-$C_4$, un groupe alcynyle en $C_2$-$C_4$, un groupe cycloalkyle en $C_3$-$C_6$, un groupe phényle ou un groupe benzyle, et

$R^5$ représente un groupe alkyle en $C_1$-$C_4$, un groupe alcényle en $C_2$-$C_4$, un groupe alcynyle en $C_2$-$C_4$, un groupe cycloalkyle en $C_3$-$C_6$, un groupe phényle ou un groupe benzyle.

**8.** Triazolinones de formule (XII)

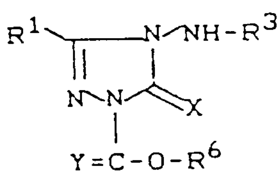

(XII)

dans laquelle

$R^1$, $R^3$, X et Y ont les significations indiquées à la revendication 1, et

$R^6$ représente un groupe alkyle en $C_1$-$C_4$, un groupe phényle ou un groupe benzyle.

**9.** 1H-triazolinones de formule (II)

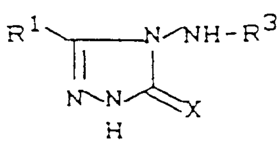

(II)

dans laquelle

$R^1$ représente un groupe éthyle, un groupe n- ou i-propyle, un groupe n-, i-, s- ou t-butyle, un groupe n- ou i-pentyle, un groupe n- ou i-hexyle, un groupe cyclopropyle, un groupe cyclopentyle ou un groupe cyclohexyle,

$R^3$ représente un groupe méthyle, et

X représente un atome d'oxygène.

**10.** 1H-triazolinones de formule (II)

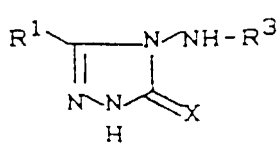

(II)

dans laquelle

- R1 représente un groupe méthyle, un groupe éthyle, un groupe n- ou i-propyle, un groupe n-, i-, s- ou t-butyle, un groupe n- ou i-pentyle, un groupe n- ou i-hexyle, un groupe cyclopropyle, un groupe cyclopentyle ou un groupe cyclohexyle,
- $R^3$ représente un groupe éthyle, un groupe n- ou i-propyle, un groupe n- ou t-butyle,
- X représente un atome d'oxygène.